# EUROPEAN PATENT APPLICATION

(11) **EP 4 534 425 A2**
(43) Date of publication of application: **09.04.2025**
(21) Application number: 23812214.7
(22) Date of filing: 26.05.2023
(51) Int. Cl.: B65B 7/16, B65B 41/06, B65B 57/04, B65H 3/08

(54) **WELL-PLATE SEALING APPARATUS**

(30) Priority: 27.05.2022 KR 20220065486; 27.05.2022 KR 20220065493; 27.05.2022 KR 20220065502; 27.05.2022 KR 20220065508; 21.02.2023 KR 20230022700; 21.02.2023 KR 20230022709
(71) Applicant: Seegene, Inc., Seoul 05548 (KR)
(72) Inventor: YUK, Nam Su, Seoul 05224 (KR); YOON, Dong Il, Suwon-si Gyeonggi-do 16543 (KR); NA, Yong Kyun, Seoul 06601 (KR); SHIN, Yung Soo, Yongin-si Gyeonggi-do 16857 (KR); PARK, In Seo, Hwaseong-si Gyeonggi-do 18506 (KR); KANG, Byung Yong, Osan-si Gyeonggi-do 18121 (KR); PARK, Jun Seong, Hwaseong-si Gyeonggi-do 18439 (KR); HAN, Jin Keun, Pyeongtaek-si Gyeonggi-do 17870 (KR); ROH, Kyoung Min, Seoul 05315 (KR); KIM, Han Kyoo, Seoul 05280 (KR); OH, Pil Seung, Suwon-si Gyeonggi-do 16507 (KR)
(74) Representative: Viering, Jentschura & Partner mbB Patent- und Rechtsanwälte
(86) International application number: PCT/KR2023/007314
(87) International publication number: WO 2023/229440

(57) **Abstract**

A well plate sealing apparatus according to the present invention comprises: a magazine for accommodating stacked sheets, a pickup unit for picking up the uppermost sheet in the magazine, a tray on which a well plate is disposed, a sheet transfer unit for transferring the picked-up sheet to a placing position, and a sealing unit for sealing wells of the well plate with the sheet, wherein the placing position is a position in which the picked-up sheet is placed on the well plate disposed on the tray.

## Description

### [Technical field]

The present invention relates to a well plate sealing apparatus.

### [Background technology]

As the interest in the health of modern people increases and the life expectancy is extended, the importance of nucleic acid-based in vitro molecular diagnosis, such as accurate analysis of pathogens and gene analysis of patients, is increasing, and the demand is increasing. Nucleic acid-based molecular diagnosis is performed by extracting nucleic acid from a sample and then checking the presence or absence of a target nucleic acid among the extracted nucleic acid.

Polymerase chain reaction (PCR) is the most widely used nucleic acid amplification reaction, and includes the processes of denaturation of doublestranded DNA, annealing of oligonucleotide primers to a DNA template, and repeated cycles of primer extension by DNA polymerase (Mullis et al., U.S. Pat. Nos. 4, 683, 195, 4, 683, 202, and 4, 800, 159; Saiki et al., (1985) Science 230, 1350-1354).

During the polymerase chain reaction detection process, a liquid sample is added to a well plate having a plurality of wells formed therein, and a film is covered and sealed on the well plate in order to seal an inlet formed on the top surface of the well plate.

In general, a film used for sealing is provided in a roll-type in which the film is continuously wound on a roller. Japanese Patent Laid-Open Publication No. 2000-335509 discloses a method for sealing a roll-type microplate and an automatic sealing apparatus for a microplate.

However, such a roll-type sealing apparatus requires a separate cutter for cutting a continuous film, and requires a plurality of guide rollers for flattening the wound film. Therefore, there is a problem that the apparatus is large and the price is high.

In order to solve this problem, a sheet-type film in which a plurality of film sheets cut to fit the width of the plate are loaded may be used.

### [Detailed description of the invention]

### [Disclosure]

### [Technical Problem]

In this background, the first to fourth exemplary embodiments of the present invention provide a well plate sealing apparatus capable of sealing a plate through an automated process while using a sheet-type film stack and preventing occurrence of an error.

According to another aspect, the fifth and sixth embodiments of the present invention provide a sheet adsorbing apparatus which may adsorb the uppermost sheet through an automation process while using a sheet-type film stack and prevents the occurrence of an adsorption error.

### [Technical solving method]

i) In order to achieve the above object, a first embodiment of the present invention may provide a well plate sealing apparatus, comprising: a magazine capable of accommodating stacked sheets; a pickup unit configured to pick up the uppermost sheet of the sheets in the magazine; a tray on which a well plate is disposed; a sheet transfer unit coupled to the pickup unit and configured to transfer the picked-up sheet to a placing position; and a sealing unit configured to seal wells of the well plate with the sheet, wherein the placing position is a position in which the picked-up sheet is placed on the well plate disposed on the tray.

Here, the wells of the well plate may be arranged in rows and columns, and the sealing unit may be configured to seal all of the wells by attaching the sheet to the well plate.

Here, the sealing unit may include a heating unit for transferring heat to the sheet covered on the well plate.

Here, the tray may include a finger member movable between an outer side and an inner side in the width direction of the well plate, and the finger member may be configured to move inward in the width direction of the well plate to prevent the sheet on the well plate from being curling up while the sheet is covered on the well plate.

Here, the finger member may be configured to move outward in the width direction of the well plate before the heating unit approaches the sheet to avoid interference with the heating unit.

Here, the finger member may include a first finger portion positioned outside in the width direction of the well plate and a second finger portion extending from the first finger portion inward in the width direction of the well plate.

Here, the apparatus may further include magazine guide rail for guiding the magazine in a direction inclined vertically, wherein the magazine descends along the magazine guide rail by gravity to be stopped at a predetermined sheet pick-up position.

Here, the apparatus may further include a controller, wherein the controller may control the apparatus to: transfer the tray on which the well plate is disposed to the placing position, pick up the uppermost sheet in the magazine using the pickup unit, and transfer the picked-up sheet to the placing position using the pickup unit, place the sheet down on the well plate at the placing position using the pickup unit, transfer the tray on which the well plate covered with the sheet is disposed to a sealing position, seal the well plate with the covered sheet at the sealing position using the sealing unit, and transfer the tray on which the well plate sealed with the sheet is disposed to an unloading position.

Here, the pickup unit may include a hand for adsorbing the sheet and at least one blower fan positioned behind the hand.

Here, the apparatus may further include a controller, and the controller may drive the blower fan so that positive pressure is generated in the hand, thereby separating the adsorbed sheet and put the separated sheet on the well plate.

Here, the pickup unit may drive the blower fan to adsorb the uppermost sheet from the magazine so that negative pressure is generated in the hand.

Here, the blower fan may include a first blower fan which is driven to generate positive pressure in the hand, and a second blower fan which is driven to generate negative pressure in the hand.

Here, the pickup unit may include a hand configured to pick up a sheet, and the sheet transfer unit may include a rotation driving part configured to drive the hand to be rotatable between the magazine and the well plate and a translation driving part configured to drive the hand to be translatable between the magazine and the well plate.

Here, the rotation driving part may be configured to rotate about a rotation axis aligned with the x-axis, and the translation driving part may include a first translation driving part configured to linearly move in the y-axis direction and a second translation driving part configured to linearly move in the z-axis direction.

Here, the pickup unit may include a hand configured to pick up a sheet, and the hand may perform a rubbing motion in a state in which the hand is in contact with the uppermost sheet to separate and pick up the uppermost sheet from the sheet located thereunder.

Here, the rubbing motion may include one or more movements in the plane direction of the sheet.

Here, the rubbing motion may include one or more movements in the plane direction of the sheet while lifting the sheet.

Here, the pickup unit may be configured to pick up the uppermost sheet among the sheets from the magazine capable of accommodating sheets on which a film sheet and a slip sheet are alternately stacked, and may include a sheet sensor capable of sensing each of the film and the slip sheet.

Here, the sheet sensor may include a first sensor configured to sense the film and a second sensor configured to sense the slip sheet.

Here, the first sensor and the second sensor may be mounted to be irradiated to the sheet at different incident angles.

According to another embodiment, there may be provided a well plate sealing apparatus, the apparatus including: a well plate loading area in which a well plate is loaded on a tray and unloaded from the tray; a sheet supply area configured to adsorb the uppermost sheet among the sheets stacked in a magazine and transfer the adsorbed sheet to supply the sheet to the well plate; a heating area configured to apply heat to the sheet covered over the well plate; and a tray transfer unit configured to transfer the tray on which the well plate is loaded along the loading area, the sheet supply area, and the heating area.

Here, the apparatus may further include a pickup unit including a hand for adsorbing the sheet and one or more blower fans positioned behind the hand and configured to supply the sheet adsorbed in the sheet supply area to the well plate.

Here, the pickup unit may perform any one or more of a first operation of driving the blower fan to separate the adsorbed sheet so that positive pressure is generated in the hand and a second operation of driving the blower fan to adsorb the uppermost sheet so that negative pressure is generated in the hand.

Here, the blower fan may include a first blower fan configured to be driven so that positive pressure is generated in the hand, and a second blower fan configured to be driven so that negative pressure is generated in the hand, and the first blower fan and the second blower fan may be disposed side by side in a direction perpendicular to the sheet.

Here, it may further include one or more blower fans for suctioning air in the sheet supply area and discharging it to the heating area.

Here, the apparatus may further include a magazine accommodating the stacked sheets and a magazine guide rail for guiding the magazine in a direction inclined vertically, wherein the magazine descends along the magazine guide rail by gravity to be stopped at a predetermined sheet pick-up position.

Here, the apparatus may further include a sheet transfer unit including a rotation driving part configured to rotatably drive a hand picking up the sheet between the magazine and the well plate, and a translation driving part configured to linearly move between the magazine and the well plate.

Here, the apparatus may further include a pickup unit configured to pick up the uppermost sheet among the plurality of sheets in a magazine capable of accommodating sheets in which film sheets and slip sheets are alternately stacked, wherein the sheet supply area may include a placing position in which the pickup unit places the film sheet down on the well plate disposed on the tray when the picked-up sheet is the film sheet, the well plate moves to the placing position and places the first sheet down on the well plate when the picked-up sheet by the pickup unit is the first sheet, and the well plate moves out of the placing position and places the second sheet down outside the well plate when the picked-up sheet by the pickup unit is the second sheet.

Here, the apparatus may further include: a tray transfer unit configured to transfer the tray; a sheet sensor configured to sense the sheet accommodated in the magazine; a well plate sensor configured to sense whether the well plate is disposed on the tray; and a control unit, wherein the control unit may: a) drive the tray transfer unit to transfer the tray to the placing position when it is determined that the well plate is disposed on the tray by receiving a signal from the well plate sensor; b) drive the pickup unit to pick up the sheet when it is determined that the sheet is accommodated in the magazine by receiving a signal from the sheet sensor; c) drive the sheet transfer unit to transfer the sheet to the placing position; d) drive the pickup unit to drop the sheet on the well plate; e) drive the tray transfer unit to move the tray to a sheet sealing area after step d); f) drive the sealing unit to seal wells of the well plate to the sheet after step e); and g) drive the tray transfer unit to transfer the tray to the loading area after step f).

Here, the pickup unit may be configured to pick up the uppermost sheet of the sheets in the magazine which is capable of accommodating sheets on which film sheets and slip sheets are alternately stacked, and further includes a sheet sensor which is capable of sensing each of the film sheet and the slip sheet, and in the step d), when it is determined that the picked-up sheet by the pickup unit is the film sheet by using a signal of the sheet sensor, the pickup unit is driven to put the film sheet down on the well plate which waits at the placing position, and when it is determined that the picked-up sheet by the pickup unit is the slip sheet by receiving the signal of the sheet sensor, the pickup unit may be driven to move the tray to a position which is out of the placing position, and the pickup unit may be driven to drop the slip sheet at the placing position.

Here, the pickup unit may include a hand configured to suction a sheet, and one or more blower fans positioned behind the hand, and the blower fan may include a first blower fan configured to drive so that positive pressure is generated in the hand, and a second blower fan configured to drive so that negative pressure is generated in the hand.

Here, the magazine may include a sheet separation part providing an external force to separate the uppermost picked-up sheet by the pickup unit among the sheets and a sheet thereunder, the sheet separation part may include a first friction member provided on an inner wall of one side of the magazine, and the hand may rub the picked-up sheet against the first friction member while rotating at a predetermined angle about a rotation axis parallel to the plane direction of the sheet in a state in which the uppermost sheet is picked up.

Here, the first friction member may be provided at one side in the longitudinal direction of the magazine, and the hand may rub the picked-up sheet against the first friction member while rotating at a predetermined angle about a rotation axis parallel to the width direction of the sheet.

Here, the magazine may include a sheet separation part providing an external force to separate the uppermost picked-up sheet by the pickup unit among the sheets and a sheet thereunder, the sheet separation part may include a second friction member provided on an inner surface of a side wall in the width direction of the magazine, and the hand may rub the picked up sheet against the second friction member while moving in the height direction having a predetermined angle in the plane direction of the sheet in a state in which the uppermost sheet is picked up.

Here, the control unit may: a) approach the sheet in a state in which the pickup surface of the hand is parallel to the stacked sheets; b) pick up the uppermost sheet when it is determined that the pickup surface of the hand is in contact with the sheet from the signal of the contact sensor; c) retreat the hand from the stacked sheets by a predetermined distance after the hand picks up the uppermost sheet; d) rotate the hand in one direction at a predetermined angle about a rotation axis parallel to the width direction of the sheet; e) rotate the hand in the reverse direction at a predetermined angle about a rotation axis to approach the sheet in a state in which the pickup surface is parallel to the stacked sheets; f) rotate the hand in the reverse direction at a predetermined angle about a rotation axis; and g) rotate the hand in one direction at a predetermined angle about a rotation axis so that the pickup surface is parallel to the stacked sheets.

Here, the magazine may include a sheet separation part configured to provide an external force to separate the uppermost picked-up sheet by the pickup unit among the sheets and a sheet thereunder, the sheet separation part may include a first friction member provided on an inner wall of one side in the longitudinal direction of the magazine, and the picked up sheet may be rubbed against the first friction member while the hand rotates at a predetermined angle about the rotation shaft.

Here, the sheet separation part may further include a second friction member provided on an inner wall of one side in the width direction of the magazine, and the control unit may allow the hand to operate at least one of the retraction and the advance by a predetermined distance from the stacked sheets after the hand picks up the uppermost sheet, so that the picked-up sheet rubs against the second friction member.
ii) In order to achieve the above object, a second embodiment of the present invention may provide a sheet transfer apparatus, including: a magazine configured to accommodate stacked sheets; a pickup unit configured to pick up the uppermost sheet of the sheets in the magazine; a tray on which an object member is disposed; a sheet transfer unit coupled to the pickup unit and configured to transfer the picked-up sheet to a placing position; and a control unit, wherein the placing position is a position at which the picked-up sheet is placed on the object member disposed on the tray, the pickup unit includes a hand configured to suction the sheet and one or more blower fans positioned behind the hand, and the control unit controls the fan to be driven so that negative pressure is generated in the hand, so that the hand holds the uppermost sheet of the sheets in the magazine.

Here, the controller may drive the blower fan to generate positive pressure in the hand, and control the hand to separate the adsorbed sheet and put the sheet on the target member.

Here, the blower fan may be provided to be rotatable in both directions.

Here, the blower fan may be provided such that a first blower fan and a second blower fan, which are different from each other, are arranged in series in the blowing direction, and the first blower fan and the second blower fan may be provided to rotate in different directions.

Here, the pickup unit may further include a contact sensor configured to sense whether the hand is in contact with the sheet, and the controller may determine information collected from the contact sensor to transmit a signal for driving the hand.

Here, the hand may include a sheet receiving surface facing the sheet, an air hole provided in the sheet receiving surface, and an airtight member provided in the sheet receiving surface and surrounding a periphery of the air hole to include the air hole therein.

Here, the airtight member may be formed with a plurality of semi-independent bubbles or independent bubbles.

Here, the airtight member may be formed of a polymer foam.

Here, the sealing member may be formed of ethylene propylene diene monomer (EPDM) foam.

Here, the hand may further include a plurality of space securing protrusions protruding forward from the sheet receiving surface on which the air hole is formed.

Here, the air hole may be disposed in plural in a circumferential direction along a wing of the blower fan, and the space securing protrusion may include a central space securing protrusion positioned at a center of the circumferential air hole and a peripheral space securing protrusion positioned around the circumferential air hole.

Here, the sheet transfer unit may include: a rotation driving part configured to drive the hand to be rotatable between the magazine and the object member; and a translation driving part configured to drive the hand to be translatable between the magazine and the object member.

Here, the rotation driver may be provided to rotate about a rotation axis along the x-axis, and the translation driver may include a first translation driver linearly moving in the y-axis direction and a second translation driver linearly moving in the z-axis direction.

Here, the hand may perform a rubbing motion in a state in which the hand is in contact with the uppermost sheet to separate and pick up the uppermost sheet from a sheet located thereunder.

Here, the rubbing motion may include one or more movements in the plane direction of the sheet.

Here, the rubbing motion may include one or more movements in the plane direction of the sheet while lifting the sheet.

Here, the pickup unit may be configured to pick up the uppermost sheet among the plurality of sheets from the magazine which is capable of accommodating sheets on which a film sheet and a slip sheet are alternately stacked, and may further include a sheet sensor which is capable of sensing each of the film sheet and the slip sheet.

Here, the sheet sensor may include a first sensor configured to sense the film sheet and a second sensor configured to sense the slip sheet.

Here, the first sensor and the second sensor may be mounted to be irradiated to the sheet at different incident angles.

Here, the target member may be a well plate in which a plurality of wells are formed, and the apparatus may further include a sealing unit configured to seal the wells of the target member with the sheet.

Here, the sealing unit may be configured to seal all of the wells by attaching the sheet to the target member.

Here, the sealing unit may include a heating unit for transferring heat to the sheet covered on the target member.

Here, the controller may automatically perform a process of putting down and sealing the picked-up sheet from the magazine on the target member on the tray.

According to another embodiment of the present invention, there may be provided a sheet transfer apparatus including: a magazine configured to accommodate stacked sheets; a pickup unit configured to pick up a uppermost sheet among the sheets in the magazine; a tray on which an object member on which the sheet put down by the pickup unit is covered is disposed; a sheet transfer unit coupled to the pickup unit and configured to transfer the picked-up sheet; and a controller, wherein the pickup unit includes a hand configured to adsorb the sheet and one or more blower fans positioned behind the hand, and the controller drives the blower fans so that positive pressure is generated in the hand to allow the hand to separate the adsorbed sheet and put the sheet down on the object member.

Here, the hand may have an adhesive member and may pick up the uppermost sheet among the sheets in the magazine by attaching the uppermost sheet.
iii) In order to achieve the above object, the third embodiment of the present invention may provide a sheet transfer apparatus, including: a magazine capable of accommodating stacked sheets; a pickup unit configured to pick up a uppermost sheet among the sheets in the magazine; and a control unit, wherein the pickup unit includes a hand configured to pick up the sheet, and the control unit controls the hand to perform a rubbing motion in a state in which the hand is in contact with the uppermost sheet to separate the uppermost sheet from a sheet located thereunder.

Here, the pickup unit may further include a contact sensor configured to sense whether the hand is in contact with the sheet, and the controller may determine information collected from the contact sensor to transmit a signal for driving the hand.

Here, the pickup unit may generate a negative pressure to adsorb the uppermost sheet among the sheets in the magazine, and the hand may include a sheet receiving surface facing the sheet, an air hole provided on the sheet receiving surface, and an airtight member provided on the sheet receiving surface and surrounding a periphery of the air hole so as to include the air hole therein.

Here, the airtight member may be formed with a plurality of semi-independent bubbles or independent bubbles.

Here, the airtight member may be formed of a polymer foam.

Here, the sealing member may be formed of ethylene propylene diene monomer (EPDM) foam.

Here, the pickup unit may include one or more blower fans positioned behind the hand, and the controller may drive the blower fans to generate negative pressure in the hand, and control the hand to adsorb the uppermost sheet of the sheets in the magazine.

Here, the air hole may be provided in a plurality of air holes arranged in a circular shape based on a wing center of the blower fan.

Here, the pickup unit may generate negative pressure to adsorb the uppermost sheet among the sheets in the magazine, and the hand may include a sheet receiving surface facing the sheet and a curve prevention member preventing the picked-up sheet from curling up.

Here, the curve prevention member may be elastically deformable, may protrude downward from the sheet receiving surface, and may extend to the outside of the sheet receiving surface.

Here, the magazine MS may include a sheet separating part SS which provides an external force to separate the uppermost sheet of the sheets ST picked up by the pickup unit GU from sheets ST disposed thereunder.

Here, the sheet separating part may include a separating wing which protrudes to an inner side surface of one side wall of the magazine.

Here, the sheet separating part may include a friction member provided at an inner side of one side wall of the magazine.

Here, a plurality of semi-independent bubbles or independent bubbles may be formed in the friction member.

Here, the friction member may be formed of a polymer foam.

Here, the friction member may be formed of ethylene propylene diene monomer (EPDM) foam.

Here, the magazine MS may include a buffering member CM on a bottom surface thereof, the buffering member CM being elastically deformable.

Here, the magazine may include an antistatic member or an antistatic member that absorbs or removes static electricity from the sheet.

Here, the rubbing motion may include one or more movements in the plane direction of the sheet.

Here, the rubbing motion may include one or more movements in the plane direction of the sheet while lifting the sheet.

Here, the magazine may include a sheet separation part providing an external force to separate the uppermost picked-up sheet by the pickup unit among the sheets and a sheet thereunder, the sheet separation part may include a first friction member provided on an inner wall of one side of the magazine, and the hand may rub the picked-up sheet against the first friction member while rotating at a predetermined angle about a rotation axis parallel to the plane direction of the sheet in a state in which the uppermost sheet is picked up.

Here, the first friction member may be provided at one side in the longitudinal direction of the magazine, and the hand may rub the picked-up sheet against the first friction member while rotating at a predetermined angle about a rotation axis parallel to the width direction of the sheet.

Here, the magazine may include a sheet separation part providing an external force to separate the uppermost picked-up sheet by the pickup unit among the sheets and a sheet thereunder, the sheet separation part may include a second friction member provided on an inner surface of a side wall in the width direction of the magazine, and the hand may rub the picked up sheet against the second friction member while moving in the height direction having a predetermined angle in the plane direction of the sheet in a state in which the uppermost sheet is picked up.

Here, the control unit may: a) approach the sheet in a state in which the pickup surface of the hand is parallel to the stacked sheets; b) pick up the uppermost sheet when it is determined that the pickup surface of the hand is in contact with the sheet from the signal of the contact sensor; c) retreat the hand from the stacked sheets by a predetermined distance after the hand picks up the uppermost sheet; d) rotate the hand in one direction at a predetermined angle about a rotation axis parallel to the width direction of the sheet; e) rotate the hand in the reverse direction at a predetermined angle about a rotation axis to approach the sheet in a state in which the pickup surface is parallel to the stacked sheets; f) rotate the hand in the reverse direction at a predetermined angle about a rotation axis; and g) rotate the hand in one direction at a predetermined angle about a rotation axis so that the pickup surface is parallel to the stacked sheets.

Here, the magazine may include a sheet separation part configured to provide an external force to separate the uppermost picked-up sheet by the pickup unit among the sheets and a sheet thereunder, the sheet separation part may include a first friction member provided on an inner wall of one side in the longitudinal direction of the magazine, and the picked up sheet may be rubbed against the first friction member while the hand rotates at a predetermined angle about the rotation shaft.

Here, the sheet separating part may further include a second friction member provided on an inner wall of one side in the width direction of the magazine, and the control unit may allow the hand to operate at least one of the retraction and the advance by a predetermined distance from the stacked sheets after the hand picks up the uppermost sheet, so that the picked-up sheet rubs against the second friction member.

iv) In order to achieve the above object, a fourth embodiment of the present invention may include a magazine configured to accommodate sheets on which film sheets and slip sheets are alternately stacked; a pickup unit configured to pick up the uppermost sheet of the sheets in the magazine; a sheet conveying unit coupled to the pickup unit and configured to convey the picked up sheet to a placing position; and a sealing unit configured to seal the wells of the well plate with the film sheet, wherein the pickup unit may include a hand configured to pick up the sheet, and a sheet sensor configured to sense each of the film sheet and the slip sheet, and the placing position may be a position where the pickup unit puts the film sheet down on the well plate disposed on the tray when the picked-up sheet is the film sheet.

Here, the sheet sensor may include a first sensor configured to sense the film sheet and a second sensor configured to sense the slip sheet.

Here, the first sensor and the second sensor may be mounted to be irradiated to the sheet at different incident angles.

Here, the sheet sensor may distinguish the film sheet and the slip sheet with one sensor.

Here, the pickup unit may further include a contact sensor configured to sense whether the hand is in contact with the sheet.

Here, the magazine MS may include a sensor for sensing whether the sheet is accommodated therein.

Here, the display apparatus may further include a tray in which a well plate is disposed, and a well plate sensor configured to sense whether the well plate is disposed on the tray.

Here, the apparatus may further include a tray transfer unit configured to transfer the tray, and a control unit, wherein the control unit may receive a signal of the well plate sensor and, when it is determined that the well plate is disposed in the tray, drive the tray transfer unit to transfer the tray to the placing position.

Here, the controller may drive the pickup unit to pick up the sheet, drive the sheet transfer unit to transfer the sheet to the placing position, drive the pickup unit to drop the film sheet on the well plate waiting at the placing position when it is determined that the picked-up sheet by the pickup unit is a film sheet by receiving a signal of the sheet sensor, drive the tray transfer unit to move the tray to a position deviated from the placing position when it is determined that the picked-up sheet by the pickup unit is a piece of paper by receiving the signal of the sheet sensor, and drive the pickup unit to drop the piece of paper at the placing position.

Here, the pickup unit may include one or more blower fans positioned behind the hand, and the blower fans may include a first fan which is driven to generate positive pressure in the hand, and a second fan which is driven to generate negative pressure in the hand.

Here, the sheet transfer unit may include a rotation driving part configured to drive the hand to be rotatable between the magazine and the well plate, and a translational driving part configured to drive the hand to be translatable between the magazine and the well plate.

Here, the rotation driver may be configured to rotate about a rotation axis along the x-axis, and the translation driver may include a first translation driver configured to linearly move in the y-axis direction and a second translation driver configured to linearly move in the z-axis direction.

Here, the hand may perform a rubbing motion in a state in which the hand is in contact with the uppermost sheet to separate and pick up the uppermost sheet from a sheet located thereunder.

Here, the rubbing motion may include one or more movements in the plane direction of the sheet.

Here, the rubbing motion may include one or more movements in the plane direction of the sheet while lifting the sheet.

v) In order to achieve the above objects, the fifth embodiment of the present invention may provide a sheet adsorbing apparatus, including: a magazine configured to accommodate sheets which are stacked; a pickup unit configured to pick up the uppermost sheet among the sheets in the magazine; and a control unit, wherein the pickup unit includes a hand configured to adsorb the sheet and a blower fan positioned behind the hand, the control unit drives the fan so that negative pressure is generated in the hand and controls the hand to adsorb the uppermost sheet among the sheets in the magazine, and the control unit receives a signal including driving information of the fan or pressure information behind the hand after driving the fan and determines whether the sheet is adsorbed to the hand.

Here, the controller may determine whether the blower fan is rotated by detecting a speed electromotive force of a driving part that rotates the blower fan.

Here, the pickup unit may further include an adsorption sensor configured to sense whether the sheet is adsorbed to the hand.

Here, the blower fan may be provided such that a first blower fan and a second blower fan, which are different from each other, are arranged in series in the blowing direction, and the first blower fan and the second blower fan may be provided to have different blowing directions.

Here, the first blower fan may generate negative pressure in the hand, the second blower fan may generate positive pressure in the hand, and the adsorption detection sensor may be a rotation sensor for detecting rotation of the second blower fan.

Here, the controller may determine that the sheet is not adsorbed to the hand when the controller detects that the second blower fan rotates at a predetermined speed or more after a predetermined time has elapsed after driving the first blower fan by receiving information from the adsorption detection sensor.

Here, the controller may determine that the sheet is adsorbed to the hand when it is sensed that the second blower fan does not rotate or rotates at a speed less than a predetermined speed after a predetermined time has elapsed after driving the first blower fan by receiving the information of the adsorption detection sensor.

Here, the pickup unit may further include a pressure sensor configured to sense pressure behind the hand, and the controller may receive information of the pressure sensor and determine that the sheet is adsorbed to the hand when pressure behind the hand rises to a predetermined pressure or more.

Here, the pickup unit may further include a pressure sensor configured to sense pressure behind the hand, and the controller may receive information of the pressure sensor and determine that the sheet is adsorbed to the hand when it is sensed that a change in pressure behind the hand is out of a predetermined pressure range.

Here, the blower fan may be provided to be rotatable in a bidirectional direction, and the controller may rotate the blower fan in one direction to generate negative pressure in the hand, and rotate the blower fan in an opposite direction to generate positive pressure in the hand.

Here, the pickup unit may further include a pressure sensor configured to sense pressure behind the hand, and the controller may receive information of the pressure sensor to determine whether the sheet is adsorbed to the hand.

Here, the hand may perform a rubbing motion in a state in which the hand is in contact with the uppermost sheet to separate and pick up the uppermost sheet from a sheet located thereunder.

Here, the rubbing motion may include one or more movements in the plane direction of the sheet.

Here, the control unit may perform the rubbing motion when it is determined that the sheet is adsorbed on the hand.

Here, it may further include a sheet transfer unit configured to transfer the picked-up sheet by the pickup unit.

Here, the controller may drive the blower fan to generate positive pressure in the hand, and control the hand to separate the adsorbed sheet and put the sheet on the target member.

Here, the hand may include one or more air holes and an airtight member surrounding the air holes so as to include the air holes therein.

Here, the airtight member may be formed with a plurality of semi-independent bubbles or independent bubbles.

Here, the hand may further include a plurality of space securing protrusions protruding forward from a sheet receiving surface on which the air holes are formed.

Here, the air holes may be disposed in plural in a circumferential direction along a wing of the blower fan, and the space securing protrusions may include a central space securing protrusion positioned at a center of the circumferential air holes and a peripheral space securing protrusion positioned around the circumferential air holes.

vi) In order to achieve the above object, a sixth embodiment of the present invention may provide a well plate sealing apparatus, comprising: a magazine capable of accommodating stacked sheets; a pickup unit configured to pick up the uppermost sheet of the sheets in the magazine; a sheet conveying unit coupled to the pickup unit and configured to convey the picked up sheet; a tray on which a well plate including a plurality of wells arranged in a first direction is disposed; a tray conveying unit including a tray conveying rail and a tray driving unit for guiding the tray in the first direction; a sealing unit configured to seal the wells of the well plate with the sheet; and a control unit, wherein the pickup unit includes a hand for picking up the sheet and a sheet sensor capable of detecting whether the sheet is present on the well plate, and the control unit: i) picks up the sheet by driving the pickup unit, ii) puts the sheet down on the well plate by driving the pickup unit and the sheet conveying unit, iii) relatively moves the pickup unit and the tray in the first direction, iv) detects whether the sheet is present at a plurality of predetermined positions by using the sheet sensor with reference to an expected well plate position on the tray conveying rail, and v) determines whether the sheet is positioned in a normal range on the well plate in the first direction through a signal of the sheet sensor.

Here, the controller may sense the sheet using the sheet sensor at each of a plurality of first sensing positions spaced apart from each other in the first direction, and determine the sheet to be in a normal state of a normal position when the number of first sensing positions at which the sheet is sensed is equal to or greater than a predetermined number, or determine the sheet to be in an abnormal state of an abnormal position when the number of first sensing positions at which the sheet is not sensed is equal to or greater than a predetermined number.

Here, the controller may drive the pickup unit in a second direction different from the first direction at the first sensing position, and sense the sheet by using the sheet sensor at each of a plurality of sensing points having different sensing distances.

Here, when the sheet is sensed at a first sensing point having a shorter sensing distance among the plurality of sensing points at any one of the first sensing positions, the controller may move the pickup unit and the tray relatively to the next first sensing position without sensing the sheet at a second sensing point having a longer sensing distance.

Here, the first sensing position may be located between a first position and a second position in the first direction at least one of a first end and a second end of the expected well plate position in the first direction, wherein the first position may be a position corresponding to one edge of the well plate when one edge of the sheet is located outside one edge of the well plate when the sheet is normally located on the well plate, and may be a position corresponding to one edge of the sheet when one edge of the sheet coincides with or is located inside one edge of the well plate when the sheet is normally located, and wherein the second position may be a position corresponding to the shortest edge of the well closest to the first position.

Here, the controller may sense the sheet using the sheet sensor at each of at least two first sensing positions between the first position and the second position at each of the first end portion and the second end portion of the expected well plate position, and may determine that the current state is an abnormal state when the sheet is not sensed at two or more first sensing positions among sensing results at least four first sensing positions.

Here, the control unit may sense the sheet by using the sheet sensor between one edge of the well plate and a well closest thereto at the first end portion and the second end portion of the expected well plate position in the first direction, respectively.

Here, the controller may sense the sheet using the sheet sensor at a second sensing position corresponding to at least one test well among the wells after the sealing process of the sealing unit.

Here, the second sensing position may be an inside of the test well or a periphery of the test well, and the controller may sense the sheet using the sheet sensor at each of the plurality of second sensing positions in the first direction, and determine that the sheet covers all the wells when the sheet is sensed at a predetermined number of second sensing positions or more.

Here, the pickup unit may be configured to pick up the uppermost sheet among sheets from the magazine which is capable of accommodating sheets on which a film sheet and a slip sheet are alternately stacked, and the sheet sensor may sense each of the film sheet and the slip sheet.

Here, the sheet sensor may include a first sensor configured to sense the film sheet and a second sensor configured to sense the slip sheet, and the first sensor and the second sensor may be mounted to be irradiated to the sheet at different incident angles.

Here, the sheet sensor may distinguish the film sheet and the slip sheet with one sensor.

Here, the pickup unit may further include a contact sensor configured to sense whether the hand is in contact with the sheet.

Here, the display apparatus may further include a well plate sensor configured to sense whether the well plate is disposed on the tray, wherein the controller may receive a signal from the well plate sensor and, when it is determined that the well plate is disposed on the tray, drive the tray transfer unit to transfer the tray.

Here, the controller may drive the sheet transfer unit to transfer the sheet to the placing position, wherein the placing position is a position where the picked-up sheet is placed down on the well plate disposed on the tray, wherein the controller receives a signal of the sheet sensor to place the film sheet on the well plate waiting for the placing position by driving the pickup unit when it is determined that the picked-up sheet by the pickup unit is the film sheet, and wherein the controller receives the signal of the sheet sensor to move the tray to a position deviated from the placing position by driving the tray transfer unit when it is determined that the picked-up sheet by the pickup unit is the slip sheet, and wherein the controller drives the pickup unit to drop the slip sheet to the placing position by driving the pickup unit.

Here, the sheet transfer unit may include a rotation driving part configured to drive the hand to be rotatable between the magazine and the well plate, and a translational driving part configured to drive the hand to be translatable between the magazine and the well plate.

Here, the rotation driver may be configured to rotate about a rotation axis along the x-axis, and the translation driver may include a first translation driver configured to linearly move in the y-axis direction and a second translation driver configured to linearly move in the z-axis direction.

### [Effects of the invention]

The sheet-type sealing apparatus according to the prior art has a problem in that process automation is stopped because a worker has to separate a piece of film from a film pile and place the film on a plate to be sealed.
i) According to the first embodiment of the present invention, it is possible to reduce a deviation due to manual work, save labor costs, and improve a process speed by automating a well plate sealing process while using a sheet-type film stack.
ii) According to the second embodiment of the present invention, it is possible to transfer the sheet while minimizing damage to the sheet by adsorbing the sheet with negative pressure using a blower fan.
iii) According to the third embodiment of the present invention, only the uppermost sheet may be separated through the rubbing motion of the hand to be received and transferred.
iv) According to the fourth embodiment of the present invention, in the sheet-type film stack, a film sheet and a slip sheet are distinguished from each other, and only the film sheet is placed on the well plate, thereby reducing a process time and reducing an occurrence of an error.
v) According to the fifth embodiment of the present invention, it is possible to prevent a problem in that the next process is performed in a state in which the uppermost sheet is not adsorbed in the sheet stack by determining whether or not the sheet is adsorbed after the blower fan is driven.
   In addition, according to an embodiment of the present disclosure, when it is determined that the sheet is not adsorbed after the blower fan is driven, the re-adsorption may be immediately performed to shorten the entire process time.
vi) According to the sixth embodiment of the present invention, it is possible to prevent a problem in that the plate is abnormally sealed by inspecting whether the sheet is placed in a normal position after the sheet is laid down on the plate.

In addition, according to an embodiment of the present disclosure, after sealing the sheet on the plate, it is possible to inspect whether all the wells are tightly moved and inform the user of an abnormal result.

It should be understood that the effects of the present invention are not limited to the above-described effects, and include all effects that can be deduced from the detailed description of the present invention or the configuration of the invention described in Claims.

### [Brief description of drawings]

FIG. 1 is a perspective view illustrating an outer appearance of a plate sealing apparatus according to an embodiment.
FIG. 2 is a perspective view illustrating a state in which a tray is located at a loading position.
FIG. 3 is a right side view of FIG. 2.
FIG. 4 is a left side view of FIG. 2.
FIG. 5 is a plan view of FIG. 2.
FIG. 6 is a perspective view illustrating a state in which a tray is located at a placing position.
FIG. 7 is a plan view of FIG. 6.
FIG. 8 is a perspective view illustrating a state in which a tray is located at a sealing position.
FIG. 9 is a perspective view illustrating a magazine according to an embodiment.
FIG. 10 illustrates a state in which the magazine is mounted on a plate sealing apparatus.
FIG. 11 is a perspective view illustrating a pickup unit according to an embodiment.
FIG. 12 is a bottom view of FIG. 11.
FIG. 13 is a perspective view illustrating a pickup unit according to another embodiment.
FIG. 14 is a perspective view illustrating a well plate and a tray according to an embodiment.
FIG. 15 is a perspective view illustrating a state in which the well plate is coupled to the tray module.
FIG. 16 is a flowchart showing a driving state of the pickup unit according to an embodiment.
FIG. 17 is a plan view illustrating a state in which a sheet is placed out of a normal position on a well plate.
FIG. 18 is a side view illustrating a state in which the position of a sheet is detected at a front end portion of a well plate.
FIG. 19 is a side view illustrating a state in which the position of a sheet is detected at a rear end portion of a well plate.
FIG. 20 is a flowchart illustrating a pre-sealing inspection sequence according to an embodiment.
FIG. 21 is a side view illustrating a state of inspecting a sealing state after sheet sealing is completed.
FIG. 22 is a flowchart illustrating an inspection sequence after sealing according to an embodiment.
FIG. 23 is a flowchart illustrating a drive shaft position calibration process.
FIG. 24 is a flowchart illustrating a sensing position calibration process.
FIG. 25 is a diagram illustrating a sheet separating process of the pickup unit according to an embodiment.

### [an aspect for the practice of the invention]

Hereinafter, the present invention will be described in detail with reference to an embodiment and exemplary drawings. These Examples are provided only for more specifically describing the present invention, and it will be apparent to those skilled in the art that the scope of the present invention is not limited by these Examples according to the gist of the present invention.

In addition, in adding reference numerals to components of each drawing, it should be noted that the same components are denoted by the same reference numerals as possible even though they are illustrated in different drawings. In addition, in describing the present disclosure, when it is determined that a detailed description of a related known configuration or function may obscure the gist of the present disclosure, the detailed description thereof will be omitted.

In addition, terms such as first, second, A, B, (a), (b), (i), and (ii) may be used herein when describing components of the present disclosure. Such terms are only for distinguishing the constituent elements from other constituent elements, and the essence, sequence, or order of the corresponding constituent elements is not limited by the terms. "connecting" or "coupling" a component to another component The present invention also relates to "connecting" or "coupling" of another component between each component, although the component may be directly connected or coupled to another component if it is stated that it is "connected" Alternatively, it may be understood that it may be "accessed".

The present invention relates to a plate sealing apparatus, which can be used in a detection apparatus for detecting a target analyte in a sample. The plate PLT may be a well plate in which a plurality of wells are arranged in rows and columns. The plate PLT may have a strip shape in which wells are arranged in a line. Hereinafter, the plate sealing apparatus includes a well plate sealing apparatus.

As used herein, a "sample" may include a biological sample (e.g., cells, tissues, and fluids from biological sources) and a non-biological sample (e.g., food, water, and soil). The biological sample may be virus, bacteria, tissue, cells, blood (e.g., whole blood, plasma, and serum), lymph, bone marrow, saliva, sputum, swab, aspiration, milk, urine, feces, eye fluid, semen, brain extract, spinal fluid, joint fluid, thymus fluid, bronchial lavage fluid, ascites, and amniotic fluid. The sample may also include natural nucleic acid molecules and synthetic nucleic acid molecules isolated from biological sources. In the present specification, the sample may include additional materials such as water, deionized water, saline, pH buffer, acidic solution, and basic solution.

A target analyte refers to an analyte to be analyzed. The analysis may mean, for example, obtaining information on the presence, content, concentration, sequence, activity, or properties of an analyte in a sample. Analytes may include a variety of substances (e.g., biological substances and non-biological substances such as compounds). Specifically, the analyte may include biological materials such as nucleic acid molecules (e.g., DNA and RNA), proteins, peptides, carbohydrates, lipids, amino acids, biological compounds, hormones, antibodies, antigens, metabolites, and cells. In the present specification, the analyte may be a nucleic acid molecule.

In addition, in the present specification, the sample may include an optical label. The optical label refers to a label that generates an optical signal according to the presence of a target nucleic acid. The optical label may be a fluorescent label. In the present specification, the fluorescent label may include any molecule known in the art.

Therefore, the target analyte detection apparatus of the present specification may be a target nucleic acid detection apparatus. The target nucleic acid detection apparatus allows a nucleic acid reaction in a sample to proceed, and through this, a target nucleic acid is detected.

A nucleic acid reaction refers to a series of physical and chemical reactions that generate a signal depending on the presence or absence of a nucleic acid of a specific sequence in a sample or the amount thereof. The nucleic acid reaction may be a reaction including binding of a specific sequence of nucleic acid to another nucleic acid or substance in a sample, and replication, cleavage, or decomposition of a specific sequence of nucleic acid in the sample. The nucleic acid reaction may be a reaction accompanied by a nucleic acid amplification reaction. The nucleic acid amplification reaction may include amplification of a target nucleic acid. The nucleic acid amplification reaction may be a reaction that specifically amplifies a target nucleic acid.

The nucleic acid reaction may be a signal-generation reaction, which is a reaction capable of generating a signal depending on the presence/absence or amount of a target nucleic acid in a sample. This signal-generation reaction may be a genetic analysis process such as PCR, real-time PCR, or microarray.

The target analyte detection apparatus according to an embodiment of the present specification may be a nucleic acid detection apparatus, and may detect a signal generated depending on the presence of a target nucleic acid. The nucleic acid detection apparatus may amplify and detect a signal along with nucleic acid amplification. Alternatively, the nucleic acid detection apparatus may amplify and detect a signal without accompanying nucleic acid amplification. Preferably, a signal is detected by accompaniment of nucleic acid amplification.

The target analyte detection apparatus according to an embodiment of the present specification may include a nucleic acid amplification apparatus. The nucleic acid amplification apparatus refers to a apparatus capable of performing a nucleic acid amplification reaction that amplifies a nucleic acid having a specific nucleotide sequence.

The apparatus for detecting a target analyte according to an embodiment may be an apparatus that performs a nucleic acid amplification reaction while accompanying a change in temperature. For example, in order to amplify a deoxyribonucleic acid (DNA) having a specific base sequence, the nucleic acid amplification apparatus may perform a denaturing step, an annealing step, and an extension (or amplification) step.

In particular, the target analyte detection apparatus according to an embodiment may be a apparatus that performs a nucleic acid amplification reaction and a reaction that generates an optical signal depending on the presence of a nucleic acid while accompanying a change in temperature and detects the generated optical signal.

The reaction vessel containing the sample may be made of a variety of materials, for example, plastic, ceramic, glass, or metal.

The reaction vessel is used to receive the sample to be analyzed and includes various types of vessels, for example, tubes, vials, strips to which a plurality of single tubes are connected, plates to which a plurality of tubes are connected, microcards, chips, cuvettes, or cartridges. Hereinafter, a well plate in which a plurality of wells are arranged in rows and columns will be described as an example of the reaction vessel.

The well plate is provided so that a well for accommodating the sample extends downward, and an opening of the well is provided at the upper portion. Also, the opening of the well plate is sealed after the sample or the reagent is loaded in the well of the well plate so that a fluid inside the well plate is prevented from leaking to the outside.

The well plate WP may be sealed with a transparent film. Thereafter, in the process of detecting the target analyte, the excitation light of the detection module should be able to enter the well through the opening, and the reflected light emitted from the sample should be able to exit through the opening.

In addition, the film may seal the well plate through thermal adhesion, or may seal the well plate using an adhesive or an adhesive film. Hereinafter, an embodiment in which the film is sealed to the well plate WP through thermal adhesion or thermal fusion will be described.

The plate sealing apparatus according to an embodiment of the present disclosure may be used after a process of loading a fluid containing a target analyte on a well plate is completed and before a process of detecting the target analyte through a detection module is performed. That is, after the opening of the well plate into which the fluid containing the target analyte is loaded is sealed with a film through the plate sealing apparatus, the well plate may be transferred to the next process, the target analyte detection process.

The plate sealing apparatus includes a apparatus for heating and fusing a plastic film or the like. Examples of the heating method include a hot plate welding machine, an impulse sealer, a hot air welding machine, and the like, which are heating methods from the outside, and a high frequency welding machine, an ultrasonic welding machine, and the like, which are heating methods of a material itself. Hereinafter, a plate sealing apparatus using a hot plate welding machine will be described as an example.

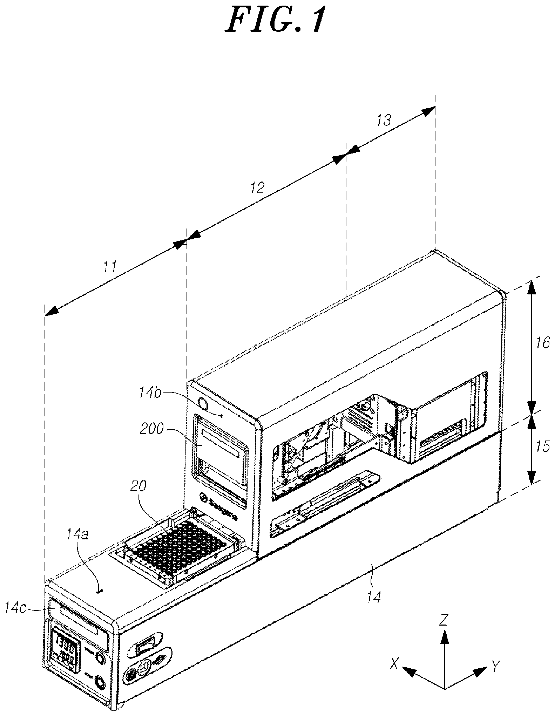

Hereinafter, a plate sealing apparatus 10 according to an embodiment will be described with reference to FIGS. 1 to 5.

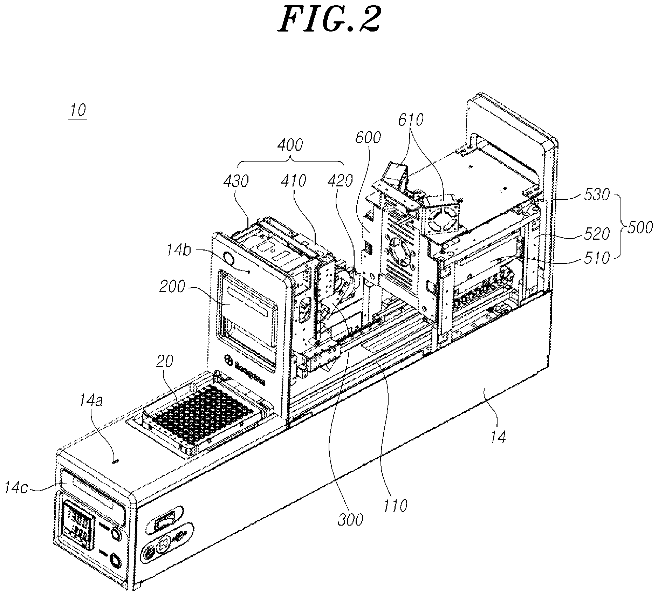

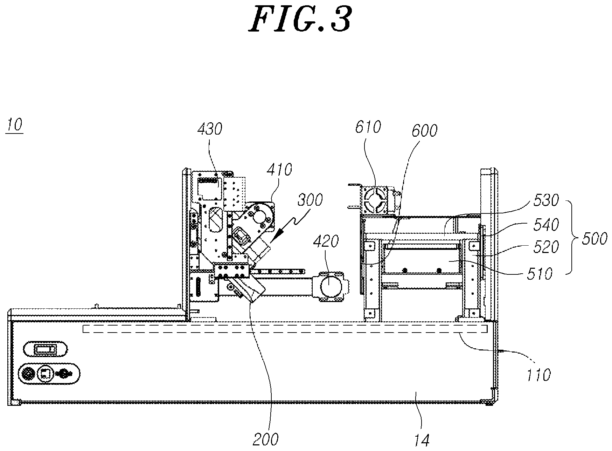

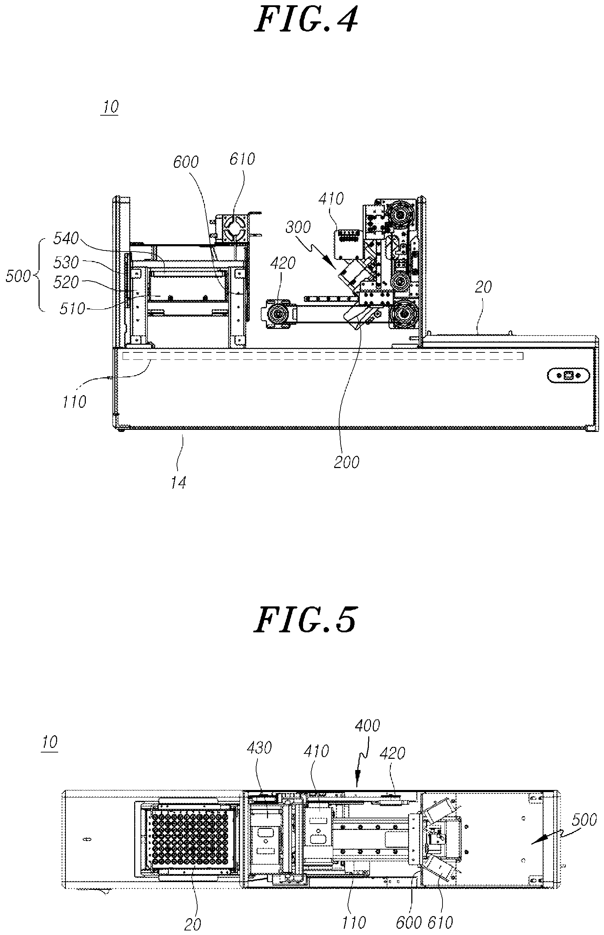

FIG. 1 is a perspective view illustrating an outer appearance of a plate sealing apparatus according to an embodiment, and FIG. 2 is a perspective view illustrating a state in which a tray is located at a loading position. FIG. 3 is a right side view of FIG. 2, FIG. 4 is a left side view of FIG. 2, and FIG. 5 is a plan view of FIG. 2.

Hereinafter, directions will be described based on the x-axis, y-axis, and z-axis of FIG. 1. However, the direction of FIG. 1 is merely an example of the plate sealing apparatus 10, and the present disclosure includes various embodiments disposed or moved in different directions.

The plate sealing apparatus 10 according to an embodiment may include a loading area 11 in which the well plate 20 in which the fluid is accommodated is loaded, a sheet supply area 12 in which the film sheet is placed on the well plate 20, and a heating area 13 in which heat is applied to the film sheet on the well plate 20 to seal the film sheet.

In addition, the loading area 11, the sheet supply area 12, and the heating area 13 are sequentially disposed in the y-axis direction, thereby reducing the process time and narrowing the width of the plate sealing apparatus 10 to improve the degree of freedom in installation.

Hereinafter, the loading position refers to a point at which the well plate 20 is loaded into the tray 100 in the loading area 11, the placing position refers to a point at which the sheet is placed on the well plate 20 in the sheet supply area 12, and the sealing position refers to a point at which the sheet is sealed to the well plate 20 in the heating area 13. The tray 100 may stop or wait for a predetermined time at the loading position, the placing position, and the sealing position.

The tray 100 on which the well plate 20 is sheeted may linearly reciprocate along the tray transfer rail 110 connecting the loading area 11, the sheet supply area 12, and the heating area 13. For example, the well plate 20 picked up by the gripper (not shown) in the loading area 11 may be sheeted on the tray 100, the film sheet may be covered on the well plate 20 after the tray 100 is moved to the sheet supply area 12 in one direction along the tray transfer rail 110, the tray 100 may be moved to the heating area 13 in one direction along the tray transfer rail 110 and then heat may be applied to the film sheet to seal the well plate 20, the tray 100 may be moved back to the loading area 11 in a reverse direction along the tray transfer rail 110, and then the gripper may pick up the well plate 20 and transfer it to the next process.

The loading area 11 may be exposed to the outside of the case 14, and the sheet supply area 12 and the heating area 13 may be accommodated in the case 14. The loading area 11 may be exposed to the outside of the case 14 to allow the gripper to enter and exit. In addition, the sheet supply area 12 and the heating area 13 may be accommodated inside the case 14 to be safe from external impact or inflow of contaminants.

Meanwhile, the tray 100 may be provided to be rotatable on an xy plane in the loading area 11. In the plate sealing apparatus 10 according to an embodiment, the well plate 20 may be transferred in a state in which the long axis of the tray 100 is disposed in the y-axis direction. When the tray 100 is provided to rotate on the xy plane in the loading area 11, the long axis of the well plate 20 may be directed in the y-axis direction while the tray 100 rotates, regardless of the direction in which the gripper loads the well plate 20.

The plate sealing apparatus 10 may include a lower area 15 and the upper area 16. A tray 100, a tray transfer rail 110, and a tray driving unit (not shown) may be provided in the lower area 15. In addition, a waste space (not shown) in which the interleaving paper to be described later may be discarded may be provided in the lower area 15.

The magazine 200, the magazine transfer rail, the pickup unit 300, the sheet transfer unit 400, and the sealing unit 500 may be provided in the upper area 16. In addition, the upper area 16 may have a smaller width in the xy plane direction than the lower area 15. Therefore, the loading area 11 may be provided outside the upper area 16.

In addition, the plate sealing apparatus 10 may be provided with a well plate detection sensor (not shown) capable of detecting whether the well plate 20 is placed on the tray 100. For example, when the well plate detection sensor detects the well plate 20, the following process may be performed. For example, the well plate detection sensor may be provided at one side of the tray 100.

In addition, the plate sealing apparatus 10 may be provided with gripper detection sensors 14a and 14b on the loading area 11. The gripper detection sensors 14a and 14b may detect a gripper or a worker's hand picking up the well plate 20 or detect the well plate 20 that is picked up. For example, the transmitter (or receiver) 14b may be provided at the upper portion of the insertion slot of the magazine 200 of the upper case 14, and the receiver (or transmitter) 14a may be provided at a front of the loading area 11 of the lower case 14 in the y-axis direction.

A partition wall 600 may be provided between the sheet supply area 12 and the heating area 13. The partition wall 600 may prevent convection between the heater 510 and the sheet supply area 12, block contaminants, and block heat transfer. In addition, the partition wall 600 may be provided above the tray transfer rail 110. That is, the tray 100 may move between the sheet supply area 12 and the heating area 13 without interference with the partition wall 600.

The sheet supply area 12 may be higher than the heating area 13 in the z-axis direction. In addition, the heat insulating plate 530 may be provided at the upper portion of the heating area 13 to block heat transfer to the upper portion. That is, the heat transfer to the sheet supply area 12 may be blocked by the partition 600 in the y-axis direction of the heating area 13 and by the insulating plate 530 in the z-axis direction.

When the heat generated in the heating area 13 is transferred to the sheet supply area 12, the degree of bending of the film sheet may become severe, and thus an error may occur in the pick-up operation or the put-down operation, and in addition, an error may occur in the sealing process.

The plate sealing apparatus 10 may further include an internal blower fan 610 provided above the insulation plate 530. The internal blower fan 610 may discharge contaminants such as dust generated in the sheet supply area 12 and simultaneously discharge hot air in the sheet supply area 12.

In addition, the plate sealing apparatus 10 may further include an external blower fan 540 provided on the rear surface of the case 14. The external blower fan 540 may discharge contaminants such as dust generated inside the case 14 and discharge hot air inside the case 14 at the same time.

Meanwhile, the film sheet may be provided in the form of a stack in which a plurality of sheets are stacked. The film sheet may have a shape corresponding to the planar shape of the well plate 20, and may have, for example, a quadrangular shape. In addition, the width of the film sheet may be provided as a width capable of covering all the wells of the well plate 20.

In addition, the sheet stack ST may be provided by alternately stacking the film sheet ST and the slip sheet ST. In order to prevent static electricity generated between film sheets overlapping each other and to prevent damage to the film sheets, a slip sheet may be interposed between adjacent film sheets.

The sheet stack may be mounted on the plate sealing apparatus 10 in a state of being accommodated in the magazine 200. For example, the magazine 200 in which the sheet stack ST is accommodated may be inserted through a slot at the upper portion of the sheet supply area 12, and may move to a sheet pickup position along the magazine transfer rail MR. Here, the sheet pick-up position refers to a point at which the pickup unit picks up a sheet in the magazine.

In this case, the slot into which the magazine 200 is inserted may be provided to be positioned above the sheet pickup position, and the magazine transfer rail may be provided to be inclined downward. When the user manually inserts the magazine 200 into the slot, the magazine 200 is moved down along the magazine transfer rail by the mass of the magazine 200 to be stopped at the sheet pick-up position. The magazine transfer rail MR. may include a catching protrusion CM at an end portion thereof. Therefore, the magazine 200 is stopped at an end portion of the magazine transfer rail.

Unlike the case in which the magazine 200 is automatically mounted, when the user manually inserts the magazine 200, it may be important whether the magazine 200 may be accurately positioned at the sheet pick-up position at every operation. If the magazine transfer rails are provided in parallel, an error may occur when the user fails to insert the magazine 200 to the sheet pickup position. However, as in the present exemplary embodiment, by providing the magazine transfer rail to be inclined downward, the magazine 200 may always reach a constant sheet pick-up position by a mass of the magazine 200 only by an operation in which the user inserts the magazine 200 into a slot.

The plate sealing apparatus 10 according to an embodiment may include a magazine 200 in which a plurality of sheets are stacked and accommodated, a pickup unit 300 configured to pick up the uppermost sheet among the plurality of sheets, a tray 100 on which a target plate on which the sheet is covered is sheeted, a tray transfer unit configured to transfer the tray 100, a sheet transfer unit 400 coupled to the pickup unit 300 and configured to transfer the received sheet to the upper portion of the tray 100, and a sealing unit 500 configured to seal the sheet on the target plate. The apparatus may further include a controller for controlling the pickup unit 300, the sheet transfer unit 400, the sealing unit 500, and the like.

The tray transfer unit may include a tray transfer rail 110 extending in the y-axis direction, and a tray driving unit (not shown) for driving the tray 100. The tray transfer rail 110 may extend to connect the loading area 11, the sheet supply area 12, and the heating area 13, and the tray driving uint may transfer the tray 100 in both directions of the y-axis.

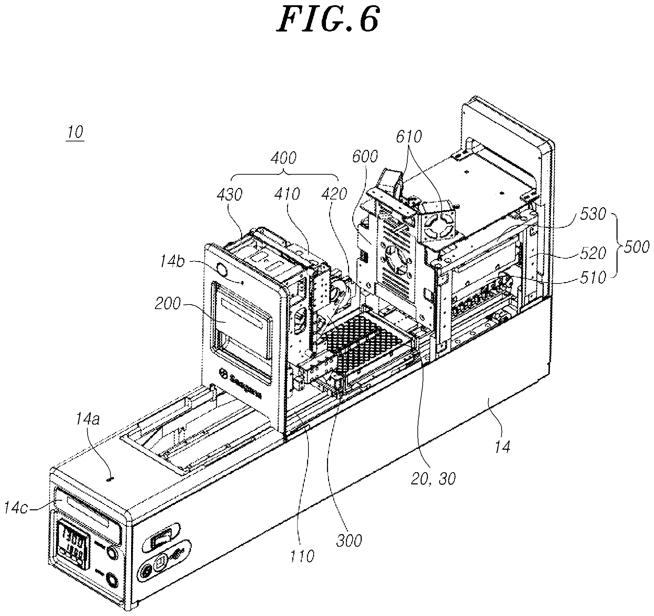

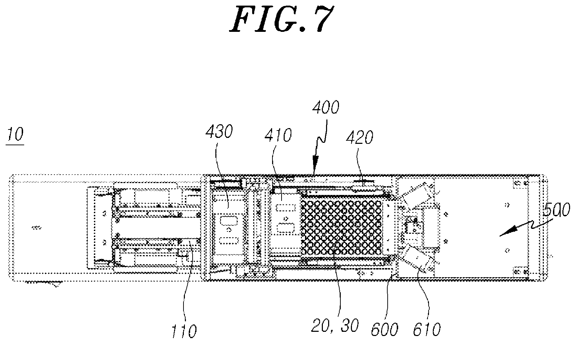

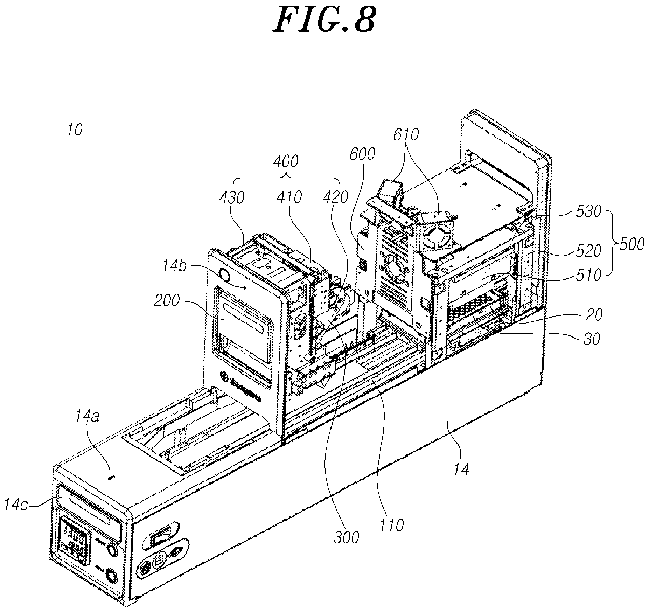

FIG. 6 is a perspective view illustrating a state in which a tray is positioned at a placing position, and FIG. 7 is a plan view of FIG. 6. FIG. 8 is a perspective view illustrating a state in which a tray is located at a sealing position.

The tray transfer unit may transfer the tray 100 to the well plate loading position in the loading area 11 to receive the well plate 20 before sealing or return the well plate 20 after sealing.

In addition, the tray transfer unit may transfer the tray 100 to the placing position in the sheet supply area 12 to receive the film on the well plate 20 from the pickup unit 300.

The tray transfer unit may transfer the tray 100 to the sealing position in the heating area 13 to seal the film on the well plate 20.

Also, the tray transfer unit may return the interleaving paper placed on the tray transfer rail 110 while returning the tray 100 to the loading position. The interleaving paper is pulled out by the tray 100 and discarded in a waist space below a loading position.

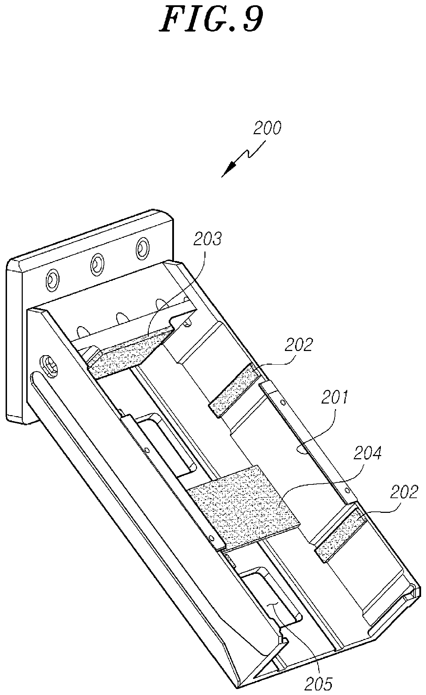

FIG. 9 is a perspective view illustrating a magazine according to an embodiment.

Referring to FIG. 9, the magazine 200 may be provided to form an accommodation space capable of accommodating the sheet stack therein, have an open upper portion, have a lower surface supporting a lower portion of the sheet stack, and have at least three side surfaces supporting a side portion of the sheet stack.

A hand may be provided in front of the y-axis of the magazine 200. The user may load the sheet stack ST after exposing the sheet stack ST to the outside of the case 14 by pulling the magazine 200 while holding the hand.

A separation wing 201 may be provided at the upper portion of a side surface of the magazine 200. The separation wing 201 is provided to protrude from the upper side of a side surface of the magazine 200 toward an inside of the magazine 200. In this case, the end of the separation wing 201 is provided to be located on the sheet. That is, when viewed from above the sheet, the end of the separation wing 201 is provided to enter the area of the sheet.

The separation wing 201 may be provided at each of both sides of the magazine 200. In this case, the distance between the separation wings 201 on both sides is provided to be smaller than the width of the sheet.

When the hand 310 picks up the sheet, the sheet is lifted while rubbing the separation wing 201. In this case, the uppermost sheet directly adsorbed on the hand 310 passes through the separation wing 201 and is lifted up, but the lower sheet not directly adsorbed on the hand 310 is dropped down by the resistance of the separation wing 201.

A friction member 202 may be provided on an inner wall of the magazine 200. Two or more friction members 202 may be provided at a distance inside one side surface of the magazine 200. The friction member 202 may be provided to be in contact with an edge of the sheet when the sheet stack is mounted in the magazine 200. Accordingly, when the hand 310 adsorbs and lifts the sheet, the edge of the sheet comes into contact with the friction member 202.

In addition, the friction member 202 may be provided to have a surface friction force. Accordingly, when the hand 310 picks up the sheet, the sheet is lifted while rubbing against the friction member 202. The uppermost sheet directly adsorbed on the hand 310 is lifted up through the friction member 202, but the lower sheet not directly adsorbed on the hand 310 is dropped down by the friction resistance of the friction member 202.

The friction member 202 may be formed of an elastically deformable material. The friction member 202 may be provided at both inner sides of the magazine 200. Therefore, the friction members 202 at both sides may align the sheet at a normal position while supporting both sides of the sheet.

For example, the friction member 202 may include a polymer foam. The polymer foam may include an ethylene propylene diene monomer (EPDM) foam.

An antistatic member 203 may be provided on an inner wall of the magazine 200. The antistatic member 203 may be provided to be in contact with the sheet at an inner side of one side of the magazine 200. Static electricity generated in the sheet may be removed through the antistatic member 203. For example, the antistatic member 203 may be an antistatic pad.

A buffer member 204 may be provided at a lower surface of the magazine 200. The buffer member 204 may be formed of an elastically deformable material. Accordingly, the buffer member 204 may partially relieve pressure applied when the hand 310 presses the sheet stack ST while supporting the lower portion of the sheet stack ST. For example, the buffer member 204 may be provided to be positioned in the middle in the longitudinal direction of the sheet stack and to extend in the width direction of the sheet stack.

For example, the friction member 202 may include a polymer foam. The polymer foam may include an ethylene propylene diene monomer (EPDM) foam.

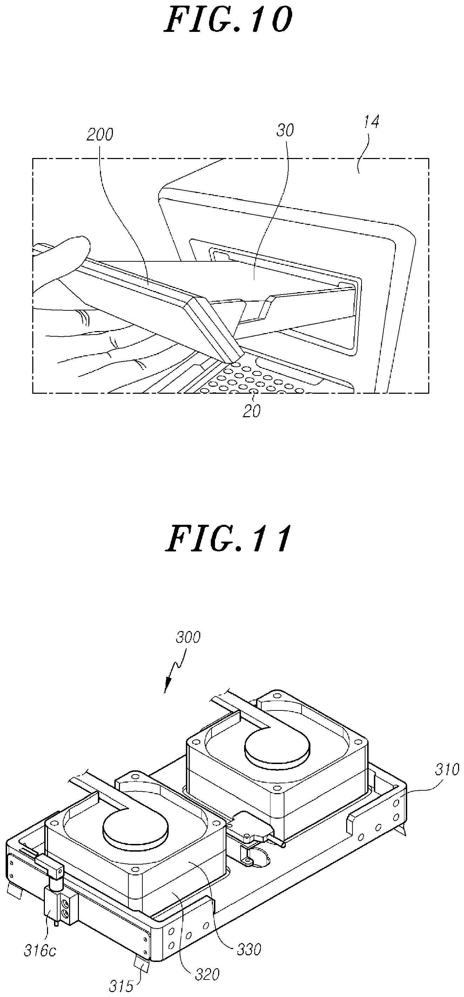

FIG. 10 illustrates a state in which the magazine is mounted on a plate sealing apparatus.

Referring to FIG. 10, the magazine transfer rail MR. may extend in the y-axis direction. At the same time, the magazine transfer rail MR. may extend in the z-axis direction. That is, the magazine transfer rail may be provided to have an inclination gradually lowered in the y-axis direction. Accordingly, when a user places the magazine 200 on the magazine transfer rail RL, the magazine 200 may be moved by its own weight along the magazine transfer rail RL. The magazine 200 may be always positioned at the same point (sheet pick-up position) as the magazine transfer rail is stopped at an end portion.

The sheet feeding unit 400 may feed the pickup unit 300 from a sheet pick-up position at which the magazine 200 is mounted to a position (a placing position) at which sheets are put down on the well plate 20. For example, the sheet feeding unit 400 may include a first translational driving part 420 that conveys the pickup unit 300 in the y-axis direction (plane direction) and a second translational driving part 430 that conveys the pickup unit 300 in the z-axis direction (height direction). For example, the translation driver may include a motor and a belt-pulley structure connected to the motor.

In addition, since the magazine transfer rail is inclined, the sheet transfer unit 400 may include a rotation driving part 410 capable of rotating the pickup unit 300 according to the inclination of the magazine transfer rail. The rotation driving part 410 may rotate the pickup unit 300 about a rotation axis along the x-axis. For example, the rotation driving part 410 may include a motor, a rotation shaft connected to the motor, and a reduction gear unit.

The pickup unit 300 may be directly connected to the rotation driving part 410, and the rotation driving part 410 may be connected to the first and second translational driving parts 430.

FIGS. 11 to 13 are views illustrating a pickup unit.

The pickup unit 300 may adsorb the uppermost sheet of the stacked sheets in the magazine 200 and may detach the adsorbed sheet. Here, "absorb" means that the uppermost sheet is attached to the hand 310 of the pickup unit 300, and includes that the sheet is attached to the hand 310 by negative pressure generated when the hand 310 sucks air. Here, "detach" includes separation by gravity after one external force that adsorbs is removed, and separation by other external force acting in a downward direction. For example, "detach" includes that the sheet is separated from the hand 310 by positive pressure formed while exhausting air in a downward direction.

The pickup unit 300 may include a hand 310 configured to pick up a sheet in the magazine 200 and a blower fan configured to provide air pressure to the sheet. The blower fan may be coupled to the rear of the hand 310. Here, the rear direction refers to a direction opposite to a direction in which the hand 310 faces the magazine 200.

The hand 310 may be positioned to face the magazine 200 at one side (front side) of the pickup unit 300, and the blower fan may be positioned to face the hand 310 at the other side (rear side) thereof. In addition, the hand 310 may include an air hole 311 for transferring air pressure provided from the blower fan. That is, the air behind the hand may be transferred to the front of the hand through the air hole 311 by positive pressure provided from the blower fan, and the air in front of the hand may be transferred to the rear of the hand through the air hole 311 by negative pressure provided from the blower fan.

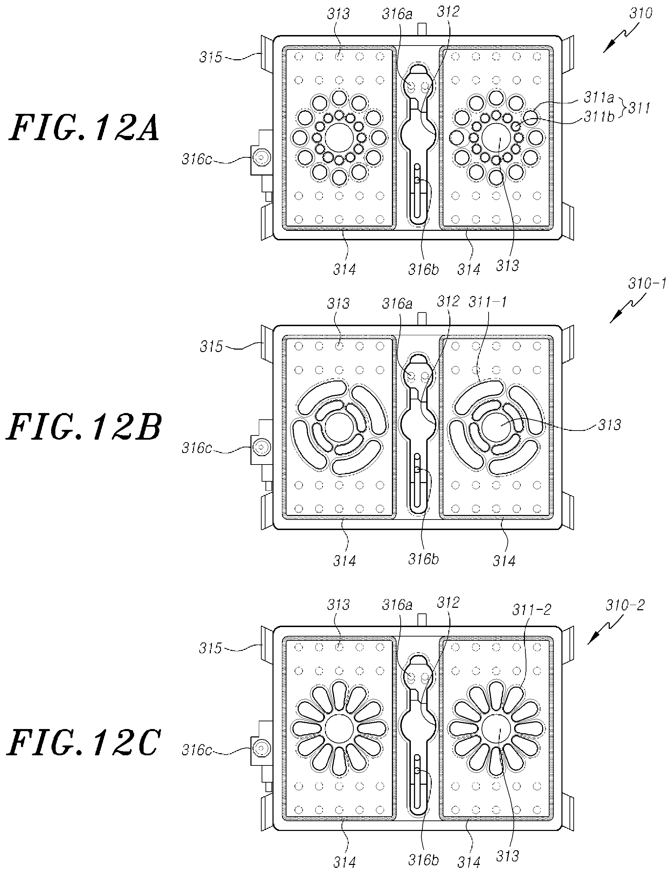

Referring to FIG. 12, the air hole 311 may include a plurality of air holes 311 disposed in a circular shape along a blade rotation trajectory of the blower fan. The air hole 311 may include an outer air hole 311a and an inner air hole 311b located inside the outer air hole 311a. The outer air hole 311a may be provided to have a larger diameter than the inner air hole 311b. The outer air hole 311a and the inner air hole 311b may be provided in the same number.

The outer air hole 311 a may be a hole through which a direct air flow passes according to rotation of the blade of the blower fan, and the inner air hole 311b may be a hole that reduces turbulence or vortex generated from the adsorption surface of the sheet or the front surface of the hand 310.

In addition, referring to FIG. 12A, the inner air holes 311b adjacent to the outer air holes 311a may be disposed in the same radial direction from the center. Alternatively, each of the inner air holes 311b may be positioned between two adjacent outer air holes 311a. In this case, a space between the outer air hole 311a and the inner air hole 311b may be minimized.

Referring to FIG. 12B, the air hole 311-1 may have a long hole shape extending in the circumferential direction along the blade rotation trajectory of the blower fan. Referring to FIG. 12C, the air hole 311-2 may have a long hole shape extending along the radial direction of the blower fan. In this case, the outer air hole and the inner air hole may be integrally formed.

In addition, the hand 310 may include a plurality of space securing protrusions 313 provided around the air hole 311. The space securing protrusion 313 may protrude from the front surface of the hand 310 toward the front of the air hole 311 to support the sheet. The space securing protrusion 313 may prevent the sheet from blocking the air hole 311. If the air holes 311 are partially blocked while the sheet is adsorbed, air pressure generated from the air holes 311 which are not blocked increases, and thus damage such as crumpling of the sheet may occur.

The space securing protrusion 313 may be provided inside and outside the air hole 311, respectively. For example, a space securing protrusion 313 may be provided in the center of the air hole 311 disposed in a circular shape, and a plurality of space securing protrusions 313 may be provided outside the air hole 311.

In addition, the pickup unit 300 may include one or more sensors 316 used to sense a sheet. The sensor 316 may include a first sensor 316a for sensing a film sheet, a second sensor 316b for sensing an interleaving paper, a third sensor 316c for sensing whether the hand 310 is in contact with the sheet, and a fourth sensor (not shown) for sensing whether the magazine 200 is empty.

Hereinafter, the first to fourth sensors may be used as terms for distinguishing sensing functions. For example, when one sensor has two or more sensing functions, it may be expressed as first or second. For example, the first sensor 316a and the second sensor 316b may be provided as one sensor. That is, one sensor may have a function of distinguishing between heterogeneous sheets.

Alternatively, the first sensor 316a and the second sensor 316b may be provided as independent sensors. In this case, the first sensor 316a and the second sensor 316b may be installed to have different incident angles with respect to a sheet plane on which the sheet is placed. Here, the incident angle refers to an angle formed by the direction of the sensor signal (wave) incident on the normal of the sheet plane.

The first sensor 316a may be installed by setting an incident angle corresponding to the film sheet, and the second sensor 316b may be installed by setting an incident angle corresponding to the interleaving paper. That is, since the film sheet and the slip sheet have different reflectivities, the first sensor 316a cannot sense the slip sheet, and the second sensor 316b cannot sense the film sheet.

The fourth sensor may be a combination of the first sensor and the second sensor. For example, when both the first sensor 316a and the second sensor 316b do not sense an object, it may be determined that the magazine 200 is empty. However, when an error occurs in the first sensor 316a or the second sensor 316b and the magazine 200 is empty, it may be erroneously determined that an object is present. In order to prevent such an error, a fourth sensor configured to detect whether the magazine 200 is empty may be further included.

The fourth sensor may be provided at an angle capable of sensing both the film sheet and the interleaving paper. A signal through hole 205 may be formed at the bottom of the magazine 200 to pass a sensor signal of a fourth sensor. Accordingly, when the magazine 200 is empty, the fourth sensor cannot sense an object.

The fourth sensor may be provided at the pickup unit 300 or may be provided below the magazine 200. In the drawing, an embodiment in which the fourth sensor IE4 is provided below the magazine 200, passes through the signal through hole 205, and senses the sheet accommodated in the magazine 200 is illustrated. Accordingly, even when the hand 310 is not positioned on the magazine 200, the fourth sensor may detect whether the magazine 200 is empty.

The pickup unit 300 may include sheet sensors 316a, 316b configured to sense a sheet. As an example, the sheet sensor SS may sense a film and a slip sheet, respectively. The sheet sensor may be provided such that one sensor may detect each of the film and the slip sheet. Alternatively, the sheet sensor may include a film detection sensor or a first sensor 316a for detecting a film, and a slip detection sensor or a second sensor 316b for detecting a slip sheet.

The blower fan may generate air pressure by rotating wings. When the wings of the blower fan rotate, positive pressure is generated on one side and negative pressure is generated on the other side. Therefore, positive pressure or negative pressure may be selectively used by changing a rotation direction or an installation position of the blade of the blower fan.

The pickup unit 300 may include a first blower fan 320 configured to generate negative pressure and a second blower fan 330 configured to generate positive pressure. The first blower fan 320 and the second blower fan 330 may be integrally provided. That is, one blower fan may be provided to change the rotation direction. Alternatively, the first blower fan 320 and the second blower fan 330 may be provided as independent structures. In this case, the first blower fan 320 and the second blower fan 330 may be installed to have different blade rotation directions.

The first blower fan 320 and the second blower fan 330 may be disposed side by side in a direction in which pneumatic pressure is generated. For example, the first blower fan 320 generating negative pressure may be disposed close to the sheet stack, and the second blower fan 330 generating positive pressure may be disposed behind the first blower fan 320. By disposing the first blower fan 320 close to the sheet stack, the intensity of the negative pressure may be increased, and the sheet may be stably adsorbed.

In addition, a plurality of blower fan stacks in which the first blower fan 320 and the second blower fan 330 are disposed to overlap each other may be disposed in the longitudinal direction of the sheet. Since the sheet is provided in a rectangular shape having different horizontal and vertical lengths, two blower fan stacks may be disposed in the vertical length direction of the sheet.

An air hole 311 corresponding to a wing of the blower fan may be formed in a front surface of the hand 310 (a surface facing the sheet), and an airtight member 314 surrounding the air hole 311 may be provided.

The airtight member 314 may include the air hole 311 and the space securing protrusion 313 therein, and may fluidly block the inside and the outside of the airtight member 314 to prevent air pressure formed through the air hole 311 from leaking to the outside of the airtight member 314. In addition, the airtight member 314 may be provided in a closed figure. A closed figure means a figure whose start point and end point coincide with each other continuously connected without interruption in the middle.

The airtight member 314 may include an independent bubble type and a semi-independent/semi-continuous bubble type. The independent bubble type is flexible, but each bubble is independent to block the fluid. In addition, in the case of the semi-independent/semi-continuous bubble type, it may be compressed with a small force, and after compression, it may be subjected to independent bubbling, thereby providing airtight performance that does not allow air or water to pass therethrough.

For example, the airtight member 314 may include a polymer foam. The polymer foam may include an ethylene propylene diene monomer (EPDM) foam.

The airtight member 314 may have a rectangular band shape including the air hole 311 and the space securing protrusion 313 therein. Alternatively, the airtight member 314 may have a circular band shape. In addition, when a plurality of blower fan stacks are provided in the longitudinal direction of the sheet, a plurality of airtight members 314 may be provided corresponding to each blower fan stack. For example, when two blower fan stacks are provided in the longitudinal direction of the sheet, two airtight members 314 having a rectangular band shape may be disposed to be spaced apart from each other.

The hand 310 may drive the first blower fan 320 to adsorb the sheet. Specifically, the hand 310 may adsorb the sheet in a state in which the airtight member 314 is in contact with or close to the sheet. More specifically, pneumatic pressure formed by driving the first blower fan 320 may be transmitted to the front of the hand 310 through the air hole 311, and the sheet may be adsorbed by negative pressure formed in the space surrounded by the airtight member 314.

The hand 310 may preserve the shape of the sheet as compared with the case of using the vacuum pump by adsorbing the sheet with the rotational force of the blower fan 320. Since the film sheet is very thin and easily wrinkled, the sheet may be damaged when negative pressure is applied to a narrow area or a vacuum pump having strong negative pressure is used. However, when negative pressure is generated inside the closed figure by using the blower fan 320 and the airtight member 314, it is possible to stably adsorb the sheet while minimizing deformation of the sheet.

Alternatively, the hand 310 may drive the second blower fan 330 to separate the sheet. Specifically, the hand 310 may drop the received sheet on the well plate 20. More specifically, positive pressure formed by driving the second blower fan 330 may be transferred to the front of the hand 310 through the air hole 311, and the sheet may be blown to be separated from the hand 310.

In the process in which the hand 310 adsorbs the sheet, since negative pressure is formed inside the airtight member 314 and the airtight member 314 blocks a fluid flow with the outside, negative pressure inside the airtight member 314 may be maintained to some extent. Therefore, even when the driving of the first blower fan 320 is stopped, the sheet may not be separated from the hand 310.

Alternatively, in the process in which the hand 310 adsorbs the sheet, an electrostatic attraction may be generated between the hand 310 and the sheet. Therefore, even when the driving of the first blower fan 320 is stopped, the sheet may not be separated from the hand 310.

In this case, by driving the second blower fan 330 to provide positive pressure to the inside of the airtight member 314, the sheet may be quickly separated.

In addition, the sheet separating process by the hand 310 may be performed in a state in which the sheet is close to or in contact with the well plate 20. If the sheet is separated from the well plate 20, the sheet may be moved left and right by the influence of the ambient air flow, and the moved sheet may not cover the well 21 formed on the upper surface of the well plate 20 or the adhesion portion may be reduced.

In addition, the hand 310 may include a sensor hole 312 in which a first sensor 316a and a second sensor 316b are installed at the center thereof and which opens signal paths of the first sensor 316a and the second sensor 316b. For example, when two blower fan stacks (units in which the first and second blower fans 320 and 330 are stacked) are provided in the longitudinal direction of the sheet, the first sensor 316a and the second sensor 316b may be spaced apart from each other in the width direction of the sheet, and the sensor hole 312 may be formed between two adjacent airtight members 314. The sensor hole 312 may have a long hole shape extending in the width direction of the sheet. For example, the first sensor 316a may be disposed on one side in the width direction of the hand 310, and the second sensor 316b may be disposed on the other side in the width direction of the hand 310. Each sensor may include a transmitter and a receiver.

In addition, a third sensor 316c may be disposed on one side of the hand 310 in the longitudinal direction. The third sensor 316c may be a contact sensor that generates a signal when coming into contact with the sheet. The third sensor 316c may be provided outside the airtight member 314.

A fourth sensor (not shown) may be disposed below the magazine 200. Specifically, the fourth sensor may be fixed to a separate frame to transmit and receive a sensing signal toward the signal through hole 205 when the magazine 200 is mounted. The fourth sensor may be provided to sense both the film and the slip sheet. However, when the magazine 200 is empty, the transmitting signal of the fourth sensor cannot detect any object.

The hand 310 may further include a curve prevention member 315. The curve prevention member 315 may prevent the sheet adsorbed on the pickup unit 300 from being bent upward.

The hand 310 may be provided to be smaller than the width of the sheet. Therefore, after the hand 310 adsorbs the sheet, the sheet may be curlled up around the hand 310. In order to prevent this, a curve prevention member 315 protruding to the outside of the hand 310 may be provided at one side of the hand 310 in the longitudinal direction.

The curve prevention member 315 may be formed of an elastic material. In addition, the curve prevention member 315 may extend further forward (in a direction facing the sheet) than the hand 310. Therefore, when the hand 310 approaches the sheet, one side of the curve prevention member 315 may contact the sheet earlier than one side of the hand 310. Alternatively, when the hand 310 approaches the sheet SH, one side of the curve prevention member 315 may first contact the sheet SH before the third sensor 316c senses the sheet SH. Alternatively, when the hand 310 approaches the sheet, one side of the curve prevention member 315 may first contact the sheet before the blower fan 320 is driven.

Thereafter, as the hand 310 continuously approaches the sheet, the curve prevention member 315 may be elastically deformed, and then the blower fan 320 may be driven to allow the hand 310 to adsorb the sheet.

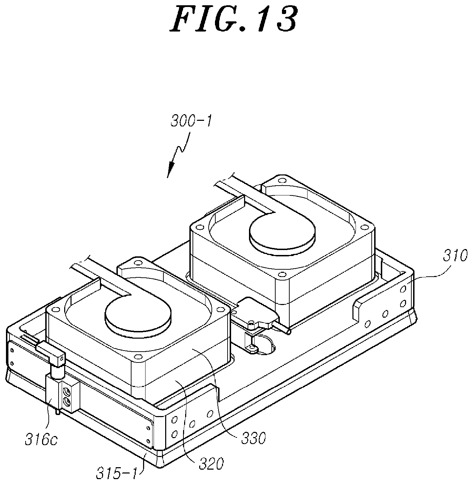

For example, referring to FIG. 11, the curve prevention members 315 may be provided at both edges in the longitudinal direction of the hand 310. Alternatively, referring to FIG. 13, the curve prevention member 315-1 may be provided at each of the four edges of the hand 310, or may be provided continuously along the four edges.

FIG. 13 is a perspective view illustrating a pickup unit according to another embodiment.

In the pickup unit 300-1 according to another embodiment, an airtight member and a curve prevention member may be integrated into one member 315-**1.** That is, the curve prevention member 315-1 continuously provided along the four edges of the hand 310 may prevent the sheet from being bent and simultaneously block the airflow between the inside and the outside of the curve prevention member 315-1. Therefore, negative pressure generated inside the curve prevention member 315-1 is maintained, so that a separate airtight member (see 314 of FIG. 12) surrounding the air hole 311 may be omitted.

The curve prevention member 315-1 may be formed of an elastically deformable material. For example, the curve prevention member 315-1 may be formed of a polymer compound, and more specifically, may be formed of a rubber material.

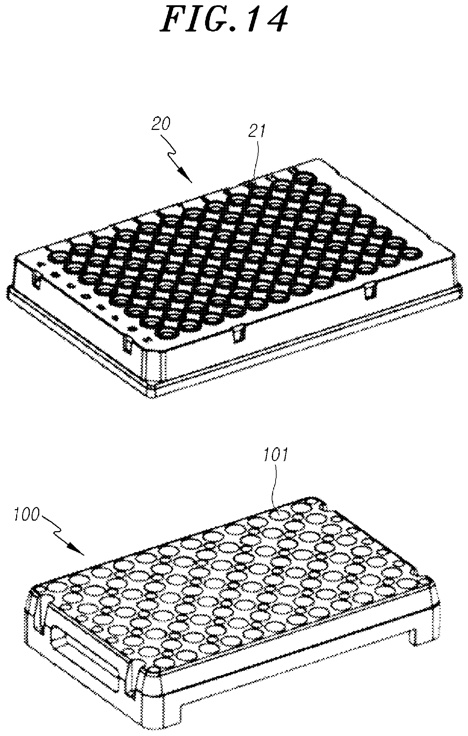

FIG. 14 is a perspective view illustrating a well plate and a tray according to an embodiment.

Referring to FIG. 14, the well plate 20 may be sheeted on the sheeting surface of the tray 100. In addition, the tray 100 may have grooves 101 formed in the sheeting surface thereof to accommodate the plurality of wells 21 protruding from the lower portion of the well plate 20. That is, the grooves 101 formed on the sheeting surface of the tray 100 are provided to correspond to the wells 21 of the well plate 20, so that the wells 21 may be inserted into the grooves 101.

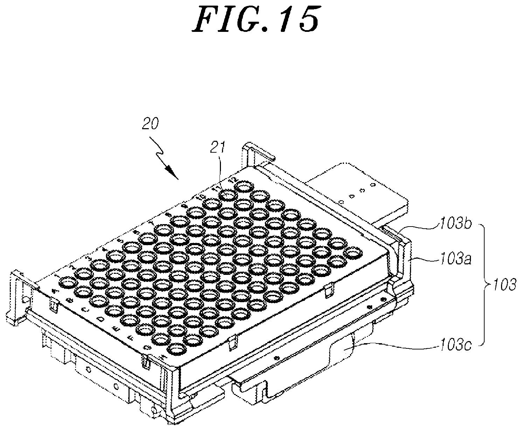

FIG. 15 is a perspective view illustrating a state in which the well plate is coupled to the tray module.

Referring to FIG. 15, the tray or the tray module 100 may further include a finger member 103. Here, the tray module 100 is used as a concept including a tray.

The finger member 103 may prevent the sheet placed on the well plate 20 from being bent or curlled upward. When the sheet on the well plate 20 is bent or curlled upward above an appropriate level, a problem may occur in the sealing process. For example, when the heater 510 of the sealing unit 500 is lowered, the bent edge of the sheet may be pushed while being in contact with the heater 510 to change the position of the sheet, or the curlled edge of the sheet may be pressed and folded by the heater 510.

The finger members 103 may be provided on both sides in the width direction of the tray 100. The finger members 103 may be located on both sides of the longitudinal edge of the tray 100. That is, the finger members 103 may be located at four edges of the tray 100, respectively. Here, the longitudinal direction may be a direction in which the length of one side of the tray 100 is longer or a movement direction of the tray 100.

The finger member 103 may include a first finger portion 103a connected to the tray 100 and a second finger portion 103b connected to the first finger portion 103a and extending inward from the outside of the tray 100 when viewed from above. The first finger portion 103a may be positioned outside the edge of the sheet when viewed from above, and the second finger portion 103b may extend from outside the edge of the sheet to inside when viewed from above.

When viewed from above, the finger member 103 may move one end of the second finger portion 103b between the inside and outside of the edge of the sheet. For example, the finger member 103 may be slidable. The first finger portion 103a may be connected to the solenoid 103c or the motor to move in both directions in a linear direction. Alternatively, the finger member 103 may pivot or hinge-move. Alternatively, the first finger portion 103a may be connected to a motor and rotationally move in both directions.

The sealing unit 500 may include a heater 510 configured to apply heat to the sheet, a heater rail 520 configured to guide vertical movement of the heater 510, a heater driver (not shown) configured to provide power for moving the heater 510, an insulating plate 530 configured to insulate the heater 510 from the upper space thereof, and an external blower fan 540 configured to discharge air heated by the heater 510 to the outside of the apparatus.

Since the sealing unit 500 may use a known product, a detailed description thereof will be omitted.

Next, an operation form of the plate sealing apparatus 10 according to an embodiment will be described.

The entire process of the plate sealing apparatus 10 may be performed in the order of waiting for a loading position of the tray 100, waiting for a sheet pickup position of the pickup unit 300, loading of the well plate 20, moving of a placing position of the tray 100, separation of sheets, pickup and transfer of sheets, placing of sheets, moving of a sealing position of the tray 100, sealing of the well plate 20, returning of a loading position of the tray 100, and unloading of the well plate 20. In addition, the process of pulling the interleaving paper in the process of returning the tray 100 to the pickup position after sealing and discarding the interleaving paper in the waste space below the loading position may be included.

The plate sealing apparatus 10 may perform a process of checking a state before performing the process of loading the well plate 20. For example, the well plate detection sensor (not shown) may detect whether the well plate 20 is placed on the tray 100, and the gripper detection sensors 14a and 14b may detect whether a gripper or another object is present on the loading area 11.

When the well plate detection sensor detects that the well plate 20 is not present, the control unit of the plate sealing apparatus 10 sends a gripper driving signal to the central control apparatus so that the gripper can perform a well plate loading operation. In addition, when the well plate detection sensor detects that the well plate 20 is placed and the gripper detection sensors 14a and 14b do not detect the object, the controller of the plate sealing apparatus 10 may transmit a signal indicating that the process is ready to the central control apparatus and perform the next process.

The tray 100 on which the well plate 20 is loaded may be moved to a placing position during separating, picking up, and transferring a sheet.

Next, when the fourth sensor detects that the magazine 200 is not empty, the sheet feeding unit 400 moves the pickup unit 300 to the sheet pickup position at which the magazine 200 is mounted in advance. Alternatively, when the fourth sensor senses that the magazine 200 is empty, the controller provides a notification that the magazine 200 is empty, and waits for the user to charge the magazine 200 with the sheet stack.

The plate sealing apparatus 10 according to an embodiment of the present disclosure may include a sheet adsorbing part. The sheet adsorbing part according to an embodiment may include a pickup unit 300 and a controller for controlling the pickup unit 300.

The pickup unit 300 may pick up the uppermost sheet among the plurality of sheets from the magazine 200 in which the plurality of sheets are stacked and accommodated. The pickup unit 300 may include a hand 310 that comes into contact with the sheet, and blower fans 320, 330 that are located behind the hand 310 and generate negative pressure in the direction of the hand. In addition, the pickup unit 300 may further include an adsorption detection sensor for detecting whether the sheet is adsorbed to the hand 310.

The controller may drive the blower fans 320 and 330 to adsorb the sheet, and after driving the blower fans 320 and 330, the controller may receive driving information of the blower fans 320 and 330 or pressure information behind the hand to determine whether the sheet is adsorbed to the hand 310. For example, the adsorption detection sensor may include a rotation sensor for detecting rotation of the blower fan, and a pressure sensor for detecting pressure behind the hand.

According to an embodiment, the controller may determine that the sheet is not adsorbed to the hand 310 when the controller detects that the second blower fan 330 rotates at a predetermined speed or more after a predetermined time has elapsed after driving the first blower fan 320 by receiving information from the adsorption detection sensor, which is a rotation sensor. Alternatively, when it is detected that the second blower fan 330 does not rotate or rotates at a speed less than a predetermined speed after a predetermined time has elapsed after driving the first blower fan 320 by receiving information from the adsorption detection sensor, the controller may determine that the sheet is adsorbed to the hand 310.

According to another embodiment, the controller may receive information of the adsorption detection sensor, which is the pressure sensor, and determine that the sheet is adsorbed to the hand 310 when pressure behind the hand rises above predetermined pressure. Alternatively, the controller may receive the information of the adsorption detection sensor and determine that the sheet is adsorbed to the hand 310 when it is detected that the pressure change behind the hand is out of predetermined pressure range.

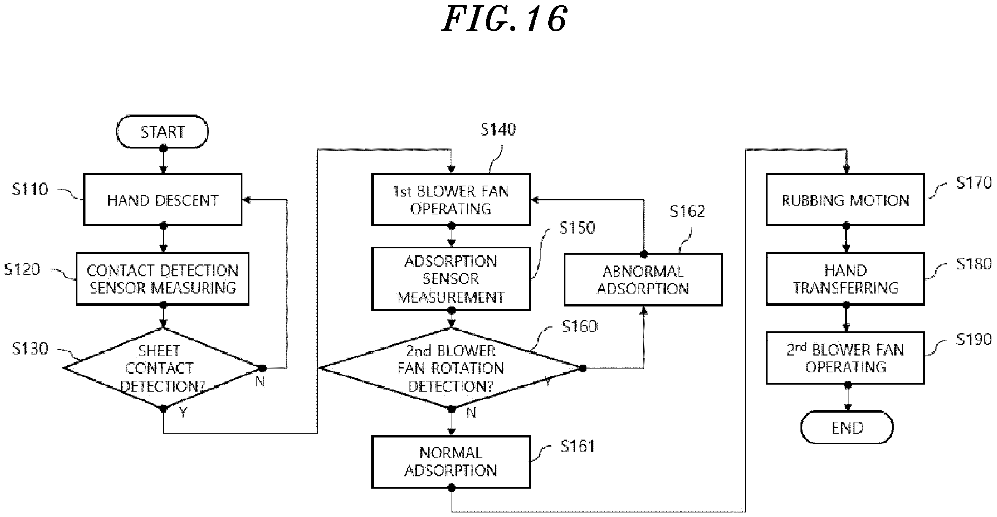

FIG. 16 is a flowchart showing a driving state of the pickup unit according to an embodiment.

Referring to FIG. 16, the controller may drive the transfer unit 400 to move the hand 310 of the pickup unit 300 in a direction close to the sheet (S110). For example, the hand 310 may move simultaneously or sequentially in the y-axis and z-axis directions, and may move forward in the y-axis direction and then descend in the z-axis direction. This is because the magazine 200 supports the sheet in a diagonal direction with respect to the z-axis direction. If the magazine 200 supports the sheet in a direction parallel to the z-axis direction, the hand 310 may descend only in the z-axis direction.

The controller may receive a signal of the third sensor 316c (contact detection sensor) (S120) to detect whether the hand 310 is in contact with the sheet (S130). The third sensor 316c may be a pressure sensor or a distance sensor. For example, the controller may receive distance information of the third sensor 316c and estimate that the hand 310 is in contact with the sheet within a predetermined distance. In this case, the predetermined distance may be a distance from the third sensor 316c to a lowermost end of the hand 310.

In addition, when the third sensor 316c detects that the hand 310 is in contact with the sheet, the controller may stop or slightly retreat the hand 310. The hand 310 may be slightly retracted to prevent a compression phenomenon between two adjacent sheets. In detail, the hand 310 may be slightly retreated to form a space under the sheet stack or between adjacent sheets, thereby preventing an air compression phenomenon between the film and the interleaving paper during the sheet separation motion of the hand 310.

Alternatively, when the third sensor 316c detects that the hand 310 is not in contact with the sheet, the controller may further lower the hand 310. That is, the controller may lower the hand 310 until the third sensor 316c senses that the hand 310 is in contact with the sheet. This is because a movement distance of the hand 310 required to contact the sheet increases as the number of sheets accommodated in the magazine 200 decreases.

Next, the control unit drives the first blower fan 320 to allow the hand 310 to adsorb the uppermost sheet (S140). Specifically, as the hand 310 comes into contact with the sheet, the airtight member 314 provided on the bottom surface of the hand 310 comes into close contact with the sheet. In addition, negative pressure is formed in an inner space of the airtight member 314 forming a closed figure (closed figure) by driving the first blower fan 320, and thus the sheet is adsorbed.

The controller may receive changed information after a predetermined time elapses after the first blower fan 320 is driven (S150) to determine whether the sheet is adsorbed to the hand 310 (S160). For example, the controller may determine whether the sheet is adsorbed to the hand 310 through the rotation speed information of the second blower fan 330.

According to an embodiment, the first blower fan 320 and the second blower fan 330 may be disposed in series with each other. In addition, the first blower fan 320 and the second blower fan 330 may be provided to have different blowing directions.

According to another embodiment, the blower fan may be provided to be rotatable in both directions, generate negative pressure in a direction of a hand by rotating the blower fan in one direction, and generate positive pressure in a direction of the hand by rotating the blower fan in an opposite direction. In this case, the pickup unit 300 may further include a pressure sensor configured to sense pressure behind the hand, and the controller may receive information of the pressure sensor to determine whether the sheet is adsorbed to the hand 310.

In an embodiment, the pickup unit 300 may be disposed close to the sheet in the order of the hand 310, the first blower fan 320, and the second blower fan 330. The first blower fan 320 rotates to provide negative pressure in front of the hand 310 to adsorb the sheet, and the second blower fan 330 rotates to provide positive pressure in front of the hand 310 to separate the sheet.

In addition, while the driving part of the first blower fan 320 is driven, driving power may not be supplied to the second blower fan 330. In addition, the second blower fan 330 may be provided in a rotatable state by an external force even when driving power is not supplied. For example, the second blower fan 330 may rotate while the air flow of the outside air passes through the second blower fan 330 at a predetermined speed or more.

When the sheet is adsorbed on the adsorption surface of the hand 310 while the first blower fan 320 is rotating, since the front opening of the hand 310 is blocked from the outside air, the air flow inside the hand 310 is reduced. In addition, when the sheet is not adsorbed to the adsorption surface of the hand 310 while the first blower fan 320 is rotating, outside air is introduced into the hand 310 through a gap between the sheet and the hand 310, and thus a rapid air flow occurs inside the hand 310.

Therefore, in the former case in which a slower air flow is formed, the second blower fan 330 rotates at a predetermined speed or less, and in the latter case in which a faster air flow is formed, the second blower fan 330 rotates over a predetermined speed. In this case, if the rotation speed information of the second blower fan 330 is known, it may be indirectly determined whether the sheet is adsorbed to the adsorption surface of the hand 310.

In an embodiment, the controller may estimate the rotation speed of the second blower fan 330 by detecting a speed electromotive force of the driver of the second blower fan 330. This is because the speed electromotive force of the rotation driving part is proportional to the rotation speed.

According to another embodiment, the controller may receive a signal from a separate rotation detection sensor capable of detecting the rotation speed of the second blower fan 330 (S150). For example, the rotation detection sensor may include a Hall sensor.

In another embodiment, the controller may receive a signal from a pressure sensor capable of sensing pressure inside the hand 310. When the sheet is adsorbed on the adsorption surface of the hand 310, pressure inside the hand 310 is instantaneously reduced, and the pressure sensor may detect this pressure change.

When it is determined that the sheet is adsorbed to the hand 310 after a predetermined time elapses after the first blower fan 320 is driven (S161), the controller may proceed with the next order, and when it is determined that the sheet is not adsorbed to the hand 310 (S162), the controller may continuously drive the first blower fan 320 (S140), or may stop the driving of the first blower fan 320 for a while and then re-drive the same. Alternatively, when it is determined that the sheet is not adsorbed to the hand 310, the controller may stop the driving of the first blower fan 320 and transmit an error message.

When it is determined that the sheet is adsorbed on the hand 310, the control unit may perform a rubbing motion. The hand 310 may perform a rubbing motion to separate and pick up the uppermost sheet from the sheet located therebelow. This rubbing motion may include one or more movements in the planar direction of the sheet.

According to an embodiment, when the sheet is normally adsorbed on the hand 310, the controller may perform a sheet separation motion of the hand 310 (S170). Since an attractive force due to static electricity may act between adjacent sheets, when the hand 310 adsorbs the uppermost sheet, another sheet may be attached and attached thereunder. Basically, the uppermost sheet and the sheet attached thereunder may be separated by using the separation wing 201 and the friction member 202 mounted on the magazine 200, but a rubbing motion the hand 310 may be performed in order to more reliably separate the uppermost sheet and the sheet.

The sheet separation motion S170 may include a rubbing motion the hand 310 in the longitudinal direction of the sheet. While the hand 310 rubs the uppermost sheet while adsorbing the uppermost sheet, shear stress is generated between the uppermost sheet and the sheet below the uppermost sheet.

The rubbing motion includes a unidirectional movement and a reciprocating movement. For example, the hand 310 may move forward in the longitudinal direction of the sheet and then pick up the sheet, or may reciprocate one or more times in the longitudinal direction of the sheet and then pick up the sheet.

The rubbing motion includes a linear movement and a curved movement. The rubbing motion includes a one-dimensional movement and a two-dimensional movement. The two-dimensional movement includes a planar movement of the sheet and a direction movement perpendicular thereto. For example, the hand 310 may move in the longitudinal direction of the sheet while drawing an arc while moving in the height direction of the sheet. In addition, the rubbing motion may include a combination of two different motions.

After the hand 310 picks up the sheet, the sheet feeding unit 400 may move the pickup unit 300 from the sheet pick-up position to the placing position at which the sheet is to be put down on the well plate 20 (S180). The sheet feeding unit 400 may move the pickup unit 300 in the y-axis direction and the z-axis direction. The sheet transfer unit 400 may include the y-axis direction driver and the y-axis direction transfer rail, and the z-axis direction driver and the z-axis direction transfer rail.

The sheet feeding unit 400 may rotate the pickup unit 300 about the rotation axis along the x-axis. The sheet feeding unit 400 may rotate the pickup unit 300 about the rotation axis along the x-axis so that the hand 310 of the pickup unit 300 faces downward.

Meanwhile, during the pick-up or during the transfer after the pick-up, the first sensor 316a and the second sensor 316b of the pickup unit 300 may detect whether the adsorbed sheet is a film or a slip sheet.

When the first sensor 316a senses the adsorbed sheet as a film, the tray driving unit moves the tray 100 to the placing position. Then, the hand 310, which has moved to the placing position, stops the first blower fan 320, and drives the second blower fan 330 to place the film on the well plate 20 (S190). Before the hand 310 puts down the film, the hand 310 may descend in the z-axis direction to move to a position where the well plate 20 and the film are close to each other. When the film is separated in a state in which the hand 310 is close to the well plate 20, an error in which the film is sheeted on the well plate 20 in a shifted state may be prevented.

Alternatively, when the second sensor 316b senses the sheet to which the sheet is adsorbed, the tray driving unit moves the tray 100 to a position past the placing position. Then, the hand 310 that has moved to the placing position stops the first blower fan 320 and drives the second blower fan 330 to place the interleaving paper on the tray transfer rail 110. Before the hand 310 puts down the interleaving paper, the hand 310 may descend in the z-axis direction to move to a position where the tray transfer rail 110 and the interleaving paper are close to each other.

Thereafter, the interleaving paper may be immediately discarded as a waste space, and may be discarded as a waste space after the sealing process. When the interleaving paper is discarded, the tray 100 may push the interleaving paper while returning to the y-axis direction, and discard the interleaving paper in the waste space provided below the well plate loading area 11. Alternatively, when moving to the waste space after the sealing process, the film may be put down on the well plate 20, and after the sealing is completed, the tray 100 may push the interleaving paper while returning to the y-axis direction to dump the interleaving paper to the waste space. In addition, the interleaving paper may be discarded through the interleaving paper outlet 14c provided in the case 14.

In addition, slide guides capable of supporting both side surfaces of the interleaving paper may be provided on the trail rail moving rails. The slide guide may extend in the y-axis direction along the trail rail and may be provided as a pair on both sides. The slide guide may guide the movement of the slip sheet to prevent the slip sheet from being inserted into a gap of the tray transfer rail 110 or being separated from the tray transfer rail 110.

After the hand 310 puts down the film on the well plate 20 that is waiting in the placing position, the finger member 103 of the tray 100 may move over the film to prevent the film from being curlled up. For example, the finger member 103 may be connected to the solenoid 103c to translate in the longitudinal direction (the y-axis direction) of the sheet. In this case, the finger member 103 may not be in contact with the film while moving.

Then, the tray 100 moves the well plate 20 to the sealing area.

The heater 510 of the sealing unit 500 is lowered to a point close to the finger member 103 along the heater rail 520. In addition, the finger member 103 may return to a position at which the film is not restrained. In addition, before the film is curlled up by the heat of the sealing area, the heater 510 descends and the film is applied with heat while being pressed on the well plate 20, so that the film may be thermally bonded or thermocompressed on the well plate 20.

After the film sealing is completed, the heater 510 ascends and the tray 100 returns to the well plate loading position. In this process, the interleaving paper on the tray transfer rail 110 is pushed by the tray 100 to move together with the tray 100, and is discarded in a waste space located outside the tray transfer rail 110. In addition, the interleaving paper may be discarded through the interleaving paper outlet 14c provided in the case 14.

Next, the control unit transmits information that the sealing is completed to the central control unit, and the gripper picks up the well plate 20 that the sealing is completed and transfers it to the next process.

Then, when the well plate detection sensor detects that the well plate 20 is not present and the gripper detection sensors 14a and 14b satisfy a condition of not detecting an object, the controller may transmit a signal indicating that the process is ready to the central control apparatus.

Next, an operation method for preventing a sealing error will be described.

The sealing error may occur in various cases. For example, a sealing error may occur when the sheet placed on the well plate 20 is not a film but a slip sheet, when the film is placed at an abnormal position shifted rather than a normal position on the well plate 20, or when the thermal adhesion surface of the film is placed on the well plate 20 and enters the sealing unit 500 in an inverted state so as not to face the well plate 20 but to face upward.

During the sealing error, when the sheet placed on the well plate 20 is not a film but a slip sheet, the sealing error may be solved by using the film detection sensor 316a and the slip detection sensor 316b. In addition, during the sealing error, when the film is placed at an abnormal position that is shifted rather than a normal position on the well plate 20, the problem can be solved by placing the sheet on the well plate 20 and then detecting the position of the sheet. Finally, during the sealing error, in the case in which the thermal adhesion surface of the film is turned upside down, not toward the well plate 20, and is placed on the well plate 20 and enters the sealing unit 500, it may be solved by checking whether a well open has occurred in the well plate 20 discharged from the sealing unit 500 after the sealing.

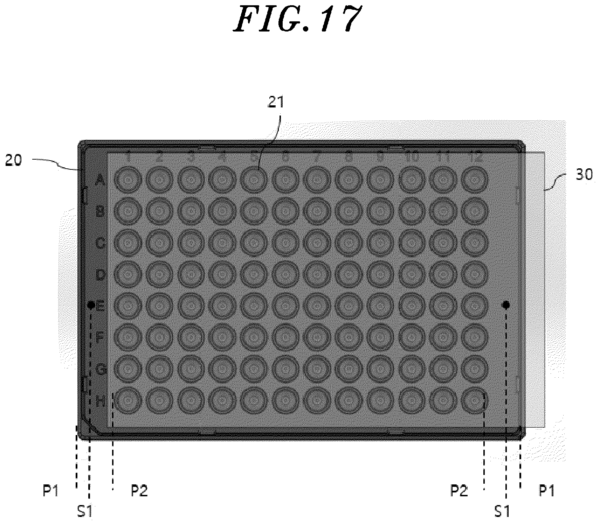

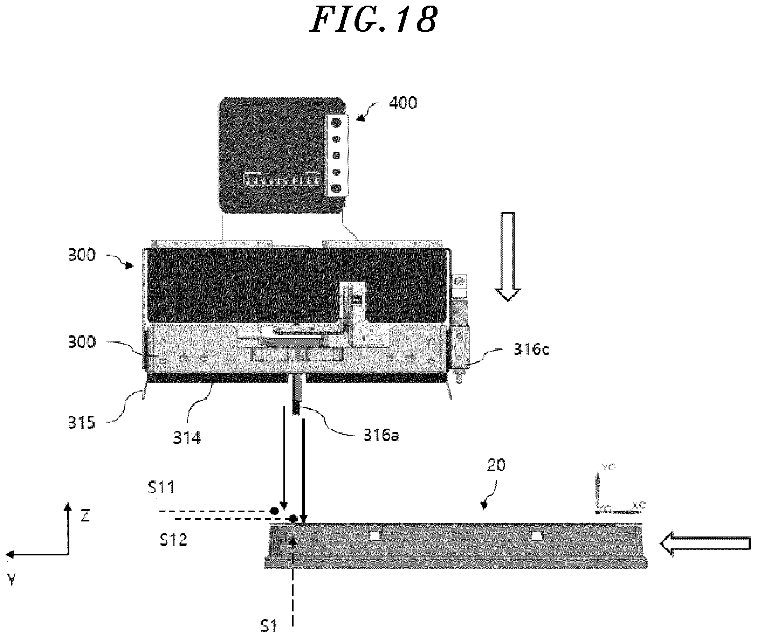

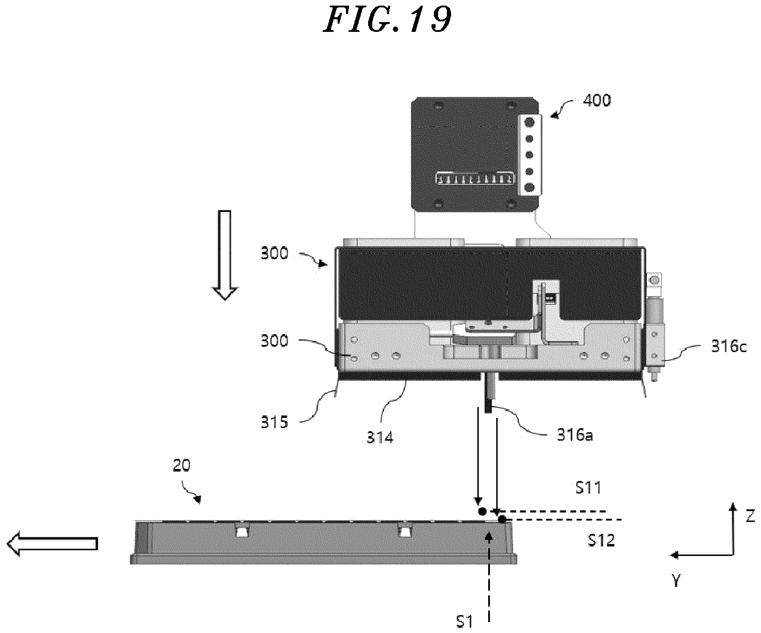

FIG. 17 is a plan view illustrating a state in which a sheet is placed out of a normal position on a well plate. FIG. 18 is a side view illustrating a state in which the location of the sheet is detected at the front end portion of the well plate, and FIG. 19 is a side view illustrating a state in which the location of the sheet is detected at the rear end portion of the well plate.

The sheet is provided to cover all of the plurality of wells 21 formed in the upper portion of the well plate 20. In addition, in consideration of the sealing stability after sealing, a sufficient space should be secured between the outermost well and the edge of the sheet. This is because an adhesive area of at least a minimum area is required for the sheet to be stably sealed around the well.

Referring to FIG. 17, the sheet is shifted to the right from the normal position on the well plate 20. Although the wells of the first row are not opened, there is a high possibility that the wells are opened when the wells are sealed in the sealing unit 500 because there is not enough space between the edge of the sheet and the first row, and even if the wells are not opened, there is a concern about sealing stability.

The drawing shows a state in which the sheet is shifted in the column direction (column 1 to column 12) of the well plate 20. Here, the column direction of the well plate 20 may be the longitudinal direction of the tray transfer rail 110, and at the same time, may be the transfer direction of the well plate 20. Alternatively, when the sheet is shifted in the row direction (row A to row H) of the well plate 20, the finger members 103 (see FIG. 15) may move the sheet to a normal position while operating. The finger member 103 may push a sheet in an abnormal position in the row direction to a normal position while being switched from an open state to a closed state.

Therefore, the controller should be able to recognize an abnormal situation when the sheet is placed to be more biased than the reference degree from the normal position in the column direction of the well 21.

The pickup unit 300 may distinguish the sheet using the sheet detection sensor 316a when picking up the sheet from the magazine 200, and may detect whether the sheet is in the correct position using the sheet detection sensor 316a even after the sheet is put on the well plate 20. For example, the sheet detection sensor 316a may be a film detection sensor (see FIG. 12) provided at a lower portion of the hand 310. In the present specification, when the sheet to be detected is a film, the sheet detection sensor may be referred to as a film detection sensor 316a, and when the sheet to be detected is a slip sheet, the sheet detection sensor may be referred to as a slip detection sensor 316b.

The sheet detection sensor 316a may be a detection sensor (e.g., an optical sensor, etc.) capable of detecting an object within a predetermined detection distance d. Therefore, in a state in which the film sheet PS is placed on the well plate 20, when the distance 11 from the sheet detection sensor 316a to the upper surface of the well plate 20 is greater than d and the distance 12 from the sheet detection sensor 316a to the upper surface of the film sheet PS is less than d, the sheet detection sensor 316a may detect only the film sheet PS. That is, the sheet detection sensor 316a may not detect an object at a point where there is no sheet on the well plate 20 due to a reason such as a shift of the film sheet, and the controller may determine that there is no sheet or the sheet is abnormally positioned.

Referring to FIGS. 17 to 19, the sheet detection sensor 316a of the pickup unit 300 may detect the position of the sheet at the first sensing position S1 of the front end portion and the rear end portion in the column direction (the first direction; the tray transfer direction; the y-axis direction) of the well plate 20. Here, the sensing of the position of the sheet includes distinguishing whether the sheet is sensed at the first sensing position S1.

In addition, a plurality of first sensing positions S1 are provided at each of the front end portion and the rear end portion of the well plate 20. According to an embodiment, the sheet detection sensor 316a may detect the presence or absence of a sheet at the first sensing location S1 of two points at each of the front end portion and the rear end portion of the well plate 20, and may detect the presence or absence of a sheet at the first sensing location S1 of a total of four points in the first direction.

The first sensing position S1 may be located between a first position P1 and a second position P2 in the first direction at least one of a first end portion (front end portion) and a second end portion (rear end portion) in the first direction of the expected well plate position. According to an embodiment, the first sensing position S1 may be located between the first position P1 and the second position P2 in the column direction in at least one of the front end portion and the rear end portion of the expected well plate position in the column direction.

Here, the first position P1 is a position corresponding to one edge of the well plate 20 in the case in which one edge of the sheet 30 is positioned outside one edge of the well plate 20 when the sheet 30 is normally positioned on the well plate 20. The first position P1 is a position corresponding to an edge of the sheet 30 when the sheet 30 is normally positioned on the well plate 20 and the edge of the sheet 30 coincides with or is positioned inside the edge of the well plate 20.

The second position P2 is a position corresponding to the shortest distance edge of the well closest to the first position P1.

Here, the expected well plate position means an expected position of the well plate 20 sheeted on the tray 100 moving along the tray transfer rail 110, and may be a position of the well plate 20 estimated through the driving of the tray driving uint. The controller may estimate the expected position of the tray through the initial position information of the tray 100 and the driving information of the tray driving unit, and may estimate the expected position of the well plate 20 through the previously known position information of the well plate 20 on the tray 100.

The expected wellplate position includes a placing position at which the tray 100 receives the sheet 30 from the pickup unit 300. In this case, in order to maintain and improve the accuracy of the expected well plate position, calibration may be periodically performed.

After the pickup unit 300 picks up the sheet from the magazine 200 and then moves to place the sheet on the well plate 20 at the placing position, the controller may relatively move the pickup unit 300 and the tray 100 in the first direction, drive the sheet detection sensor 316a when the expected well plate position is the predetermined position to determine whether the sheet is detected, and determine whether the sheet is in the normal position range on the well plate 20 through the detected information.

Here, the meaning that the pickup unit 300 and the tray 100 relatively move in the first direction includes a case in which the pickup unit 300 moves in the first direction in a state in which the tray 100 is stopped, a case in which the tray 100 moves in the first direction in a state in which the pickup unit 300 is stopped, or a case in which the tray 100 and the pickup unit 300 move in opposite directions in the first direction.

The controller may drive the sheet detection sensor 316a at a plurality of first sensing positions S1 spaced apart from each other in the first direction to determine whether the sheet is sensed, and may determine that the sheet is in a normal state of a normal position when the number of first sensing positions at which the sheet is sensed is equal to or greater than a predetermined number, or may determine that the sheet is in an abnormal state of an abnormal position when the number of first sensing positions at which the sheet is not sensed is equal to or greater than a predetermined number.

Specifically, the controller may detect the sheet by driving the sheet detection sensor 316a at the first sensing positions S1 of at least two points between the first position P1 and the second position P2 at each of the first end (the front end in the first direction) and the second end (the rear end in the first direction) of the expected well plate position, and may determine that the sheet is in the abnormal state when the sheet is not detected at the first sensing positions or more of two points among the detection results of the first sensing positions S1 or more of at least four points.

According to an embodiment, when the sheet detection sensor 316a does not detect a sheet at the first sensing location S1 of two points located at the front end and the rear end of the first end or the second end of the expected well plate location, or when the sheet detection sensor 316a does not detect a sheet at one first sensing location among the first sensing locations S1 of two points located at the first end of the expected well plate location and the sheet detection sensor 316a does not detect a sheet at one first sensing location among the first sensing locations S1 of two points located at the second end of the expected well plate location, the controller may determine that the vehicle is in an abnormal state.

In addition, the controller may drive the pickup unit 300 in a second direction different from the first direction at any one first sensing position S1 and drive the sheet detection sensor 316a at a plurality of sensing points having different sensing distances from each other to determine whether the sheet is sensed.

The sensing point refers to a point of a maximum sensing distance at which the sheet detection sensor 316a may detect a sheet, and since the maximum sensing distance of the sheet detection sensor 316a is predetermined, the sensing point may vary as the pickup unit 300 moves. The sensing point may be positioned between the shortest distance at which the sheet can be sensed and the distance at which the well plate 20 is sensed, and is positioned on the well plate 20.

The controller may relatively move the pickup unit 300 and the tray 100 to the next first sensing location S1 without sensing the sheet at the second sensing point S12 having a sensing distance longer than that when the sheet is sensed at the first sensing point S11 having a sensing distance shorter than that among the plurality of sensing points at any one first sensing location S1.

According to an embodiment, first and second sensing points S12 having different heights may be provided at any one first sensing position S1. The pickup unit 300 may drive the sheet detection sensor 316a at the first sensing point S11 having a high height among the two sensing points provided at the first sensing position S1, and when the sheet is not detected, may move downward in the z-axis direction to drive the sheet detection sensor 316a at the second sensing point S12 having a low height. Alternatively, when the sheet is detected by driving the sheet detection sensor 316a at the first sensing point S11 having a high height among the two sensing points provided at the first sensing position S1, the pickup unit 300 does not move downward in the z-axis direction but moves in the y-axis direction to move to the next first sensing position S1.

Meanwhile, in FIGS. 18 and 19, the first sensing point S11 and the second sensing point S12 are displayed at different positions in the first direction (y-axis direction), but this is for easy display in the drawings, and the first sensing point S11 and the second sensing point S12 may be located on the same first sensing position S1 in the first direction.

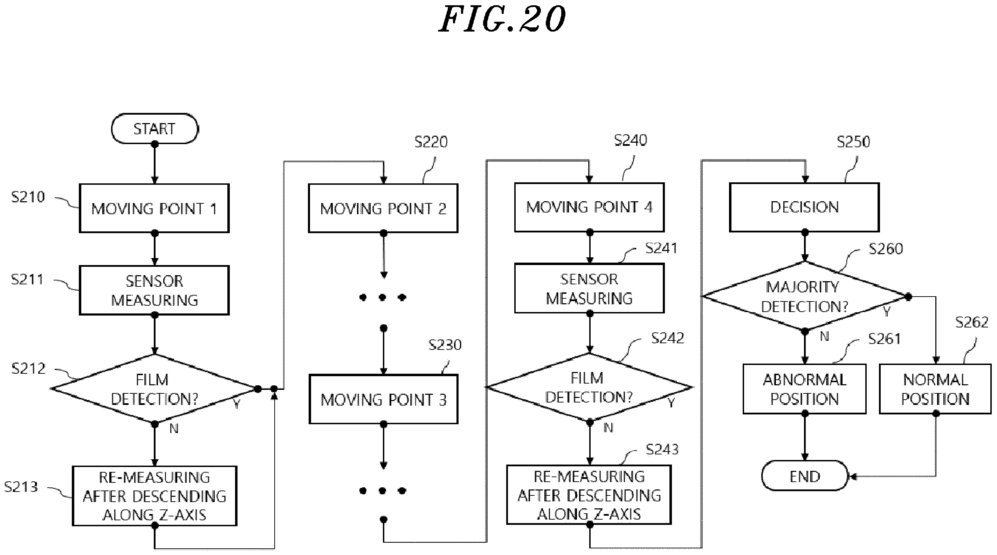

FIG. 20 is a flowchart illustrating a pre-sealing inspection sequence according to an embodiment.

Referring to FIG. 20, when it is determined that the first point RP1 among the plurality of first sensing positions S1 of the well plate 20 is positioned below the sheet detection sensor in operation S210, the controller detects the sheet through the sheet detection sensor in operations S211 and S212. In this case, the pickup unit 300 is positioned so that the sensing distance of the sheet detection sensor corresponds to the first sensing point S11.

When the sheet is sensed at the first sensing point S11, the controller moves the well plate 20 or the pickup unit 300 to a second point (S220), and when the sheet is not sensed, the controller remeasures the pickup unit 300 by moving the pickup unit 300 downward by a predetermined distance in the z-axis (S213). In this case, the pickup unit 300 moves downward so that the detection distance of the sheet detection sensor corresponds to the second sensing point S12.

The controller determines that the sheet is sensed at point 1 when the sheet is sensed at the second sensing point S12, and determines that the sheet is not sensed at point 1 when the sheet is not sensed at the second sensing point S12.

The controller repeats the operation process from point 1 to point 4 while moving the well plate 20 or the pickup unit 300.

When it is determined that point 4 among the plurality of first sensing positions S1 of the well plate 20 is positioned below the sheet detection sensor (S240), the controller detects the sheet through the sheet detection sensor (S241, S242). In this case, the pickup unit 300 is positioned so that the sensing distance of the sheet detection sensor corresponds to the first sensing point S11.

The controller completes the operation when the sheet is sensed at the first sensing point S11, and remeasures the pickup unit 300 downward by a predetermined distance in the z-axis when the sheet is not sensed (S243). In this case, the pickup unit 300 moves downward so that the detection distance of the sheet detection sensor corresponds to the second sensing point S12.

The controller determines that the sheet is sensed at point 4 when the sheet is sensed at the second sensing point S12, and determines that the sheet is not sensed at point 4 when the sheet is not sensed at the second sensing point S12.

After the sheet detection is completed at points 1 to 4 of the first sensing position S1, the controller determines whether the sheet is at the normal position (S250 and S260). In detail, when a sheet is sensed at two or more points of the first sensing positions S 1 of a total of four points, it is determined that the sheet is at a normal position (S 262), and when a sheet is sensed at less than two points of the first sensing positions S 1 of a total of four points, it is determined that the sheet is at an abnormal position (S 261). In addition, when it is determined that the sheet is in an abnormal position, an error alarm may be provided to the user.

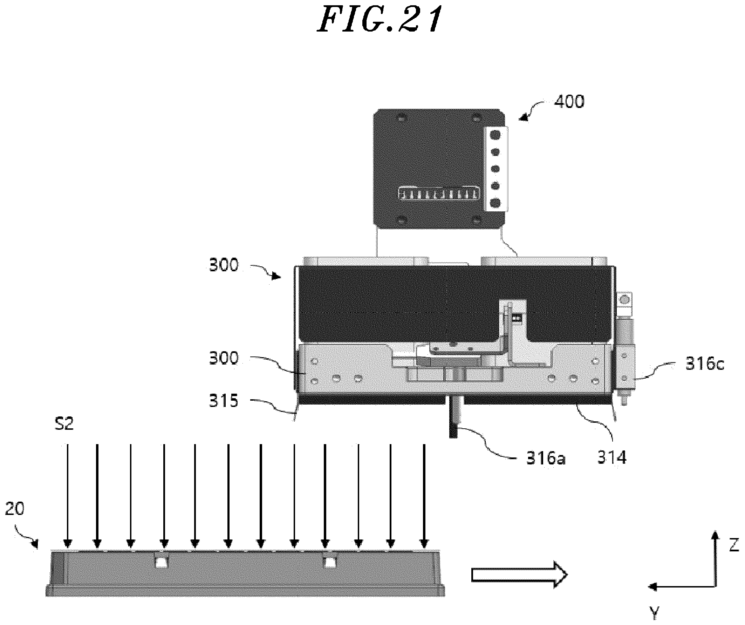

FIG. 21 is a side view illustrating a state of inspecting a sealing state after sheet sealing is completed.

The sheet detection sensor 316a of the pickup unit 300 may detect the presence or absence of a sheet at the plurality of second sensing positions S2 arranged in the column direction (the first direction; the tray transfer direction; the y-axis direction) of the well plate 20. According to an embodiment, the sheet detection sensor 316a may detect the presence or absence of a sheet at the second sensing location S2 of 12 points arranged side by side in the column direction of the well plate 20.

The second sensing location S2 may be located between the first end (front end) and the second end (rear end) in the first direction of the expected well plate location. According to an embodiment, the second sensing locations S2 may be arranged at equal intervals in the column direction of the expected well plate location.

The second sensing position S2 may be a position corresponding to the plurality of wells disposed in any one column direction of the well plate 20. Here, the positions corresponding to the plurality of wells include not only the inside of the hole of the well but also the periphery of the hole. According to an embodiment, the second sensing position S2 may be around a hole spaced apart from a plurality of wells disposed in any one column direction by the same distance.

After the well plate 20 on the tray 100 is returned to the placing position after completing the sealing process in the sealing unit 500, the controller may determine whether the sheet is sensed by driving the sheet detection sensor 316a at a predetermined position based on the expected well plate position on the tray transfer rail 110, and determine whether the sheet normally seals the wells of the well plate 20 through the sensed information.

The controller may determine whether the sheet is sensed by driving the sheet sensor 316a at a plurality of second sensing positions S2 spaced apart from each other in the first direction, and may determine that the sheet is in a normal sealed state when the number of second sensing positions S2 at which the sheet is sensed is equal to or greater than a predetermined number, or may determine that the sheet is in an abnormal sealed state when the number of second sensing positions S2 at which the sheet is not sensed is equal to or greater than a predetermined number.

Specifically, the controller may detect the sheet by driving the sheet detection sensor 316a at the second sensing location S2 of at least four points between the first end (the front end in the first direction) and the second end (the rear end in the first direction) of the expected well plate location, and may determine the state as an abnormal state when the sheet is not detected at one second sensing location S2.

According to an embodiment, when the sheet detection sensor 316a does not detect a sheet even at one of the second sensing locations S2 of 12 points located between the first end portion and the second end portion of the expected well plate location, the controller may determine the sheet as an abnormal state.

The number and positions of the second sensing positions S2 may vary. For example, the sheet detection sensor 316a may detect the sheet detection location at the second sensing locations S2 of six points while skipping, one by one, 12 wells disposed in the first direction. In addition, it may be sensed by the sheet detection sensor 316a two or more times at one second sensing position S2. For example, when the film is not sensed at the second sensing position S2, it may be sensed once more at the same height, or it may be sensed again by lowering the height of the pickup unit 300. In addition, the determination criterion of the abnormal state may also vary. For example, when a sheet is not sensed at the second sensing position S2 adjacent to each other, the controller may determine the abnormal state.

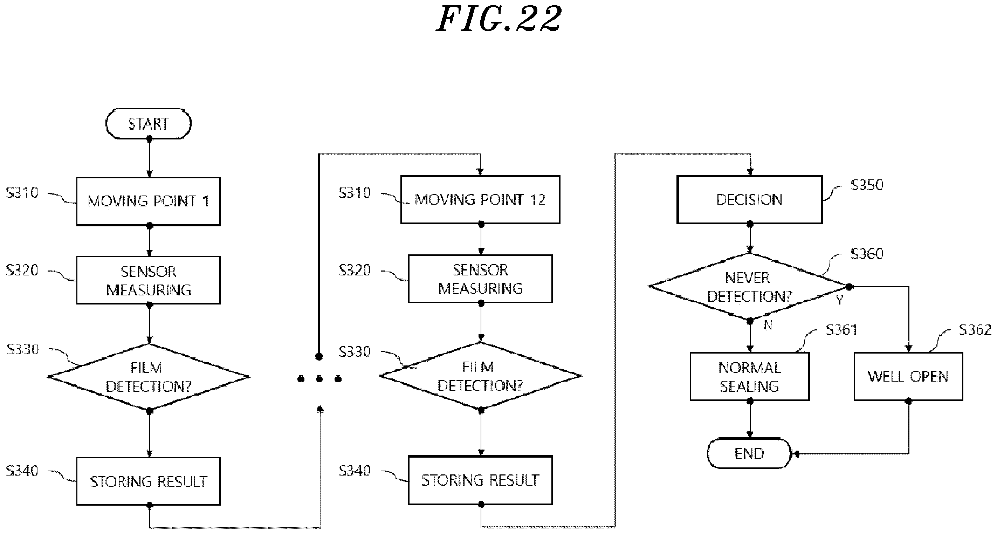

FIG. 22 is a flowchart illustrating an inspection sequence after sealing according to an embodiment.

Referring to FIG. 22, when it is determined that the first point among the plurality of second sensing positions S2 of the well plate 20 is positioned below the sheet detection sensor (S310), the controller detects the sheet through the sheet detection sensor (S320, S330). In addition, the control unit stores a result of whether a sheet is detected at a first point of the second sensing position S2 (S340).

The controller repeats the operation process from point 1 to point 12 while moving the well plate 20 or the pickup unit 300.

After the sheet sensing is completed at points 1 to 12 of the second sensing position S2, the controller determines whether a well open has occurred in the sheet (S350 and S360). Specifically, when a sheet is detected at all of the second sensing locations S 2 of a total of 12 points, it is determined that the sheet is normally sealed without a well open (S 361), and when a well open is detected at any one of the second sensing locations S 2 of a total of 12 points, it is determined that the sheet is abnormally sealed (S 362). In addition, when it is determined as abnormal sealing, an error alarm may be provided to the user.

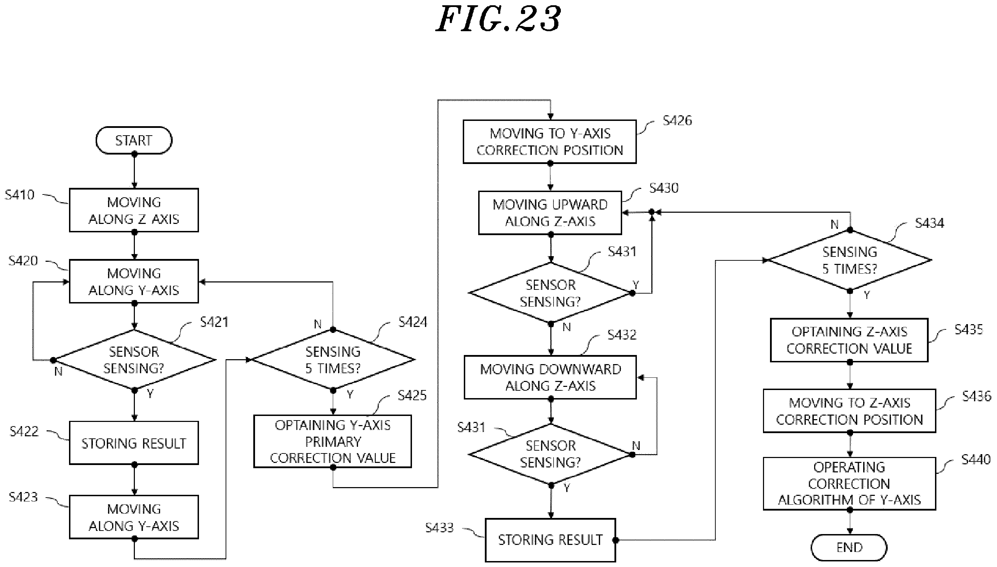

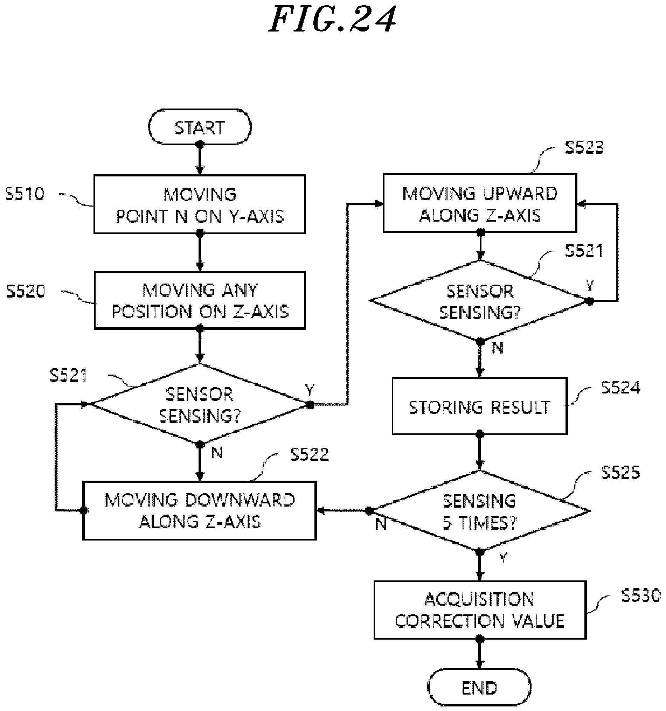

Next, a calibration process for improving sensing accuracy will be described with reference to FIGS. 23 and 24. The calibration includes a driving shaft position calibration process of adjusting the position of the driving shaft of the tray transfer unit for moving the tray 100 and the sheet transfer unit 400 for transferring the pickup unit 300, and a sensing position calibration process of adjusting the position of the sensor.

FIG. 23 is a flowchart illustrating a drive shaft position calibration process.

In order to stably perform the film normal position detection function and the well open detection function, a calibration function of equipment for the sensing position may be required. Since the deviation of the measurement position between the equipment occurs due to the component tolerance and assembly deviation of the equipment, the position of the drive shaft needs to be calibrated. The driving shaft may include the y-axis direction driving shaft for transferring the tray 100 in the y-axis direction, and the z-axis direction driving shaft for transferring the pickup unit 300 in the z-axis direction.

According to an embodiment, the tray 100 may include an origin measurement reference that may be detected by the sheet detection sensor 316a of the pickup unit 300. For example, the origin measurement criterion may be provided adjacent to an area in which the well plate 20 is mounted, and the height of the upper surface may be provided to be equal to or slightly higher than the height of the upper surface of the well plate 20 when the well plate 20 is mounted.

If a user's calibration operation is input, the pickup unit 300 is moved to an arbitrary z-axis position (S410). Then, a correction algorithm of the y-axis drive shaft is performed.

A correction algorithm of the drive axis in the y-axis direction is as follows. The pickup unit 300 is moved to an arbitrary position on the tray transfer rail 110 (S420). Until the sheet detection sensor 316a of the pickup unit 300 detects the origin measurement reference of the tray 100 (S421), the tray 100 is moved in the y-axis direction (S420). When the sheet detection sensor 316a of the pickup unit 300 detects the origin measurement reference of the tray 100 (S421), the position of the tray 100 in the y-axis direction at the corresponding time point is stored (S422). The pickup unit 300 is moved to an arbitrary position outside the tray transfer rail 110 (S423).

After repeating the above process five times (S424), the average of three values excluding the maximum value and the minimum value is used as the y-axis primary correction value (S425). Then, the tray 100 is moved to the y-axis position according to the y-axis primary correction value (S426). Then, a correction algorithm for the z-axis drive shaft is performed.

A correction algorithm of the drive shaft in the Z-axis direction is as follows.

Until the sheet detection sensor 316a of the pickup unit 300 cannot detect the origin measurement reference of the tray 100, the pickup unit 300 is moved out of the detection distance in the z-axis direction (S430). The sheet detection sensor 316a of the pickup unit 300 moves the pickup unit 300 in the z-axis direction until the origin measurement reference of the tray 100 is detected (S431) (S432). When the sheet detection sensor 316a of the pickup unit 300 detects the origin measurement reference of the tray 100 (S431), the z-axis direction position of the pickup unit 300 at the corresponding time point is stored (S433).

After repeating the above process five times (S434), the average of three values excluding the maximum value and the minimum value is used as the z-axis correction value (S435). Then, the pickup unit 300 is moved to the z-axis direction position according to the z-axis correction value (S436).

The correction algorithm of the y-axis drive shaft is performed again based on the correction value (S440).

The pickup unit 300 is moved to an arbitrary position on the tray transfer rail 110 (S420). Until the sheet detection sensor 316a of the pickup unit 300 detects the origin measurement reference of the tray 100 (S421), the tray 100 is moved in the y-axis direction (S420). When the sheet detection sensor 316a of the pickup unit 300 detects the origin measurement reference of the tray 100 (S421), the position of the tray 100 in the y-axis direction at the corresponding time point is stored (S422).

After repeating the above process five times (S424), the average of three values excluding the maximum value and the minimum value is used as the y-axis secondary correction value. Then, the tray 100 is moved to a position in the y-axis direction according to the y-axis secondary correction value.

FIG. 24 is a flowchart illustrating a sensing position calibration process.

Even after calibration of the drive shaft position is performed, a deviation may occur in the z-axis sensing area for each apparatus. For example, the sensing distance may vary due to a difference in the height or flatness of the tray 100.

According to an embodiment, the sensing position calibration may be performed immediately before the equipment is shipped and when the component is moved or reassembled, and may be performed in a state in which the well plate 20 is sheeted on the tray 100.

When a user's calibration operation is input, the tray 100 is moved by a predetermined distance based on the position in the y-axis direction according to the y-axis secondary correction value obtained through the driving shaft position calibration algorithm (S510). In this case, the moving positions are the first sensing positions S1 of four points to be used for sensing the normal position of the sealing film.

A correction algorithm of the sensing position in the z-axis direction is as follows.

The pickup unit 300 is moved to an arbitrary z-axis direction position (S520). If the well plate 20 is not sensed (S521), the pickup unit 300 moves downwardly along the z-axis until the well plate 20 is sensed (S522). If the well plate 20 is sensed (S521), the pickup unit 300 moves in the z-axis direction until the well plate 20 is not sensed (S523). If the well plate 20 is no longer sensed (S521), the z-axis direction position of the pickup unit 300 at the corresponding time point is stored (S524).

After repeating the above process five times (S525), the average of three values excluding the maximum value and the minimum value is used as the z-axis correction value (S530).

The above algorithm is repeated at each of the first sensing locations S1 of four points.

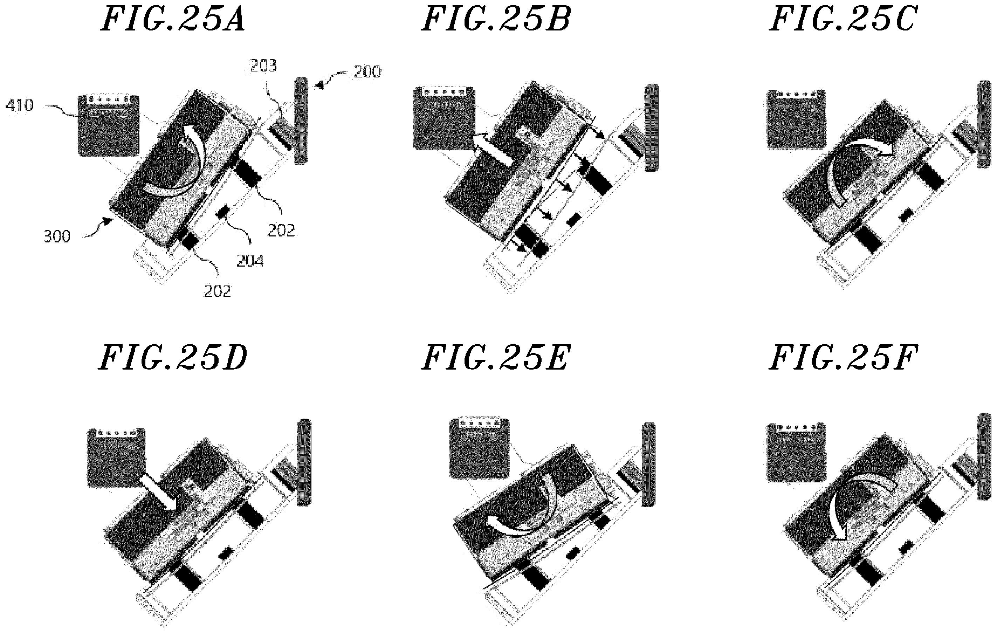

FIG. 25 is a diagram illustrating a sheet separating process of the pickup unit according to an embodiment.

In the case of a sheet stack in which a film and a slip sheet are sequentially stacked, static electricity may be generated between the film and the slip sheet during manufacturing and storage. When static electricity is generated between the film and the interleaving paper, when the hand 310 adsorbs the uppermost sheet, a heterogeneous sheet may be attached and attached thereto.

The means for separating sheet 201, 202, and 203 and the sheet separating motions of the hand 310 for preventing the aforementioned problems have been described above. Hereinafter, the sheet separation motion will be described in more detail below.

In general, static electricity between the film and the interleaving paper is less than 2 kV, and in this case, the sheet can be separated only by the linear movement of the hand. However, in some cases, static electricity of about 3 kV may be generated, and in this case, the attraction between the film and the slip sheet is strong, such that a sheet shift in which the sheet slides on the adsorption surface of the hand 310 may occur.

Specifically, when the hand 310 moves in the plane direction of the sheet in a state in which it adsorbs the uppermost sheet, static electricity between the adsorbed uppermost sheet and the sheet below the uppermost sheet acts as attraction, and thus, slippage occurs between the uppermost sheet and the adsorption surface of the hand 310. As a result, a shift occurs in the alignment of the position of the sheet adsorbed on the hand 310.

The sheet adsorbed to the hand 310 in a shifted state is placed on the well plate 20 at the placing position, and thus the same amount of shift occurs in the relative position between the ground sheet and the well plate 20. In this state, when the well plate 20 is sealed in the sealing unit 500, a sealing failure may occur.

The hand 310 of the pickup unit 300 may perform any one or more of a rubbing motion in the plane direction of the sheet, a rubbing motion in a direction perpendicular to the plane direction of the sheet, and a rubbing motion in a rotating direction rotating along a rotation axis parallel to the plane of the sheet. The hand 310 of the pickup unit 300 may perform the sheet separation motion only by a planar rubbing motion or a vertical rubbing motion. Alternatively, the hand 310 of the pickup unit 300 may perform the sheet separation motion only by a rubbing motion in the rotating direction. In some cases, the sheet separation motion may be performed by using both the horizontal rubbing motion and the vertical rubbing motion and the rotational rubbing motion.

As the hand 310 moves in a direction perpendicular to the plane direction of the sheet or rotates along a rotation axis parallel to the plane direction of the sheet, the sheet adsorbed on the hand 310 may collide with the inner wall of the magazine 200 or may be bent or curved while rubbing against the friction member 202 or the antistatic member 203. In this process, a space may be generated between the uppermost sheet adsorbed on the hand 310 and the sheet attached thereunder, and static electricity may be removed.

In addition, the hand 310 of the pickup unit 300 may repeat the same rubbing motion several times or combine different rubbing motions to perform the sheet separation motion.

Next, a sheet separating process of the pickup unit 300 according to an embodiment of the present invention will be described with reference to FIG. 25.

The pickup unit 300 lowers the hand 310 toward the sheet, and when the contact detection sensor 316c detects that the hand 310 is in contact with the sheet, drives the blower fan 320 to adsorb the uppermost sheet. The pressure between the sheets is relieved by being stepped back by a predetermined offset distance.

Next, the pickup unit 300 moves the hand 310 backward by a predetermined distance from the sheet. This is to secure a movement space of the hand 310 when a rubbing motion in the rotating direction is performed thereafter. That is, the distance by which the hand 310 retreats from the sheet may vary depending on the rotation angle of the hand 310 thereafter.

The pickup unit 300 performs a sheet separation motion.

Referring to FIG. 25A, the pickup unit 300 rotates the hand 310 in one direction about a rotation axis parallel to the plane direction of the sheet. Specifically, as the hand 310 rotates in one direction, a front edge of the sheet adsorbed on the hand 310 rubs against the antistatic member 203 located in front of the magazine 200. In this process, static electricity may be removed as the sheets that are stuck to each other are separated at the front edge of the sheet.

Referring to FIG. 25B, the pickup unit 300 moves the hand 310 backward. In the process in which the hand 310 retreats, static electricity at a side edge of the sheet may be removed while the sheet adsorbed on the hand 310 rubs against the friction member 202 formed on the side inner wall of the magazine 200.

Referring to FIG. 25C, the pickup unit 300 may rotate the hand 310 in the reverse direction to make the adsorption surface parallel to the sheet surface.

Referring to FIG. 25D, the pickup unit 300 may move the hand 310 forward toward the magazine 200. In this case, a moving distance by which the hand 310 moves forward may be smaller than a distance by which the hand 310 moves backward in FIG. 25B.

Referring to FIG. 25E, the pickup unit 300 may separate the rear edge of the sheet by rotating the hand 310 in the reverse direction.

Referring to FIG. 25F, the pickup unit 300 may rotate the hand 310 in one direction to make the adsorption surface parallel to the sheet surface.

If the uppermost sheet is not separated from the sheet attached thereunder, the sheet separation motion may be repeated. As the operation is repeated, the distance between the hand 310 and the sheet stack increases, and the rotation angle of the hand 310 may increase as much.

This patent application claims priority in Korean Patent Application Nos. 10-2022-0065508, 10-2022-0065486, 10-2022-0065493 and 10-2022-0065502 filed on May 27, 2022, and Korean Patent Application Nos. 10-2023-0022700 and 10-2023-0022709 filed on February 21, 2023, and all relevant matters are incorporated into this patent application by reference.

The above description is merely illustrative of the technical idea of the present disclosure, and those skilled in the art to which the present disclosure pertains will be able to make various modifications and variations without departing from the essential quality of the present disclosure.

Therefore, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

### [a description of a code]

10: Plate sealing apparatus, 11: loading area, 12: sheet supply area, 13: heating area, 14: case, 15: lower area, 16: upper area,
20: well plate, 21: well, 30: sheet,
100: tray, 101: groove, 103: finger member, 103a: first finger portion, 103b: second finger portion, 103c: solenoid, 110: tray transfer rail,
200: magazine, 201: separation wing, 202: friction member, 203: antistatic member, 204: buffer member, 205: signal through hole,
300: pickup unit, 310: hand, 311: air hole, 312: sensor hole, 313: space securing protrusion, 314: airtight member, 315: curve prevention member, 316a: first sensor (film detection sensor), 316b: second sensor (slip detection sensor), 316c: third sensor (contact detection sensor),
320: fist blower fan, 330: second blower fan,
400: sheet conveying unit, 410: rotation driving part, 420: first translational driving part (y-axis direction driving part), 430: second translational driving part (z-axis direction driving part),
500: sealing unit, 510: heater, 520: heater rail, 530: insulation plate, 540: external blower fan,
600: partition wall, 610: internal blower fan.

## Claims

1. A well plate sealing apparatus, the apparatus comprising:
a magazine configured to accommodate stacked sheets;
a pickup unit configured to pick up the uppermost sheet of the sheets in the magazine;
a tray on which a well plate is disposed;
a sheet transfer unit coupled to the pickup unit and configured to transfer the picked-up sheet to a placing position; and
a sealing unit configured to seal wells of the well plate with the sheet,
wherein the placing position is a position in which the picked-up sheet is placed on the well plate disposed on the tray.

2. The well plate sealing apparatus according to claim 1,
wherein the wells of the well plate are arranged in rows and columns, and
wherein the sealing unit is configured to seal all of the wells by attaching the sheet to the well plate.

3. The well plate sealing apparatus according to claim 1,
wherein the sealing unit comprises a heating unit for transferring heat to the sheet covered on the well plate.

4. The well plate sealing apparatus according to claim 3,
wherein the tray comprises a finger member movable between an outer side and an inner side in the width direction of the well plate, and
wherein the finger member is configured to move inward in the width direction of the well plate to prevent the sheet on the well plate from curling up while the sheet is covered on the well plate.

5. The well plate sealing apparatus according to claim 4,
wherein the finger member is configured to move outward in the width direction of the well plate before the heating unit approaches the sheet to avoid interference with the heating unit.

6. The well plate sealing apparatus according to claim 4,
wherein the finger member comprises a first finger portion located outside in the width direction of the well plate and a second finger portion extending from the first finger portion inward in the width direction of the well plate.

7. The well plate sealing apparatus according to claim 1,
further comprising a magazine guide rail for guiding the magazine in a direction inclined vertically, wherein the magazine descends along the magazine guide rail by gravity and stops at a predetermined sheet pick-up position.

8. The well plate sealing apparatus according to claim 1,
further comprising a controller,
wherein the controller controls the apparatus to: transfer the tray on which the well plate is disposed to the placing position, pick up the uppermost sheet in the magazine using the pickup unit, transfer the picked-up sheet to the placing position using the pickup unit, place the sheet down on the well plate at the placing position using the pickup unit, transfer the tray on which the well plate covered by the sheet is disposed to a sealing position, seal the well plate with the covered sheet at the sealing position using the sealing unit, and transfer the tray on which the well plate sealed by the sheet is disposed to an unloading position.

9. The well plate sealing apparatus according to claim 1,
wherein the pickup unit comprises a hand for adsorbing the sheet and at least one blower fan positioned behind the hand.

10. The well plate sealing apparatus according to claim 9,
further comprising a controller,
wherein the controller controls the blower fan to generate positive pressure in the hand, thereby separating the adsorbed sheet and placing the separated sheet on the well plate.

11. The well plate sealing apparatus according to claim 9,
wherein the pickup unit drives the blower fan to generate negative pressure in the hand, thereby adsorbing the uppermost sheet from the magazine.

12. The well plate sealing apparatus according to claim 9,
wherein the blower fan comprises a first blower fan configured to generate positive pressure in the hand and a second blower fan configured to generate negative pressure in the hand.

13. The well plate sealing apparatus according to claim 1,
wherein the pickup unit comprises a hand configured to pick up a sheet, and
wherein the sheet transfer unit comprises a rotation driving part configured to drive the hand to be rotatable between the magazine and the well plate, and a translation driving part configured to drive the hand to be translatable between the magazine and the well plate.

14. The well plate sealing apparatus according to claim 13,
wherein the rotation driving part is configured to rotate about a rotation axis aligned with the x-axis, and
wherein the translation driving part comprises a first translation driving part configured to linearly move in the y-axis direction and a second translation driving part configured to linearly move in the z-axis direction.

15. The well plate sealing apparatus according to claim 1,
wherein the pickup unit comprises a hand for picking up a sheet, and the hand performs a rubbing motion in a state in which the hand is in contact with the uppermost sheet to separate the uppermost sheet from the underlying sheet and pick it up.

16. The well plate sealing apparatus according to claim 15,
wherein the rubbing motion comprises at least one movement in a planar direction of the sheet.

17. The well plate sealing apparatus according to claim 15,
wherein the rubbing motion comprises at least one movement in a planar direction of the sheet while lifting the sheet.

18. The well plate sealing apparatus according to claim 1,
wherein the pickup unit is configured to pick up the uppermost sheet among the sheets from the magazine which is configured to accommodate sheets on which film sheet and slip sheet are alternately stacked, and comprises at least one sheet sensor for sensing each of the film sheet and the slip sheet.

19. The well plate sealing apparatus according to claim 18,
wherein the sheet sensor comprises a first sensor configured to sense the film sheet and a second sensor configured to sense the slip sheet.

20. The well plate sealing apparatus according to claim 19,
wherein the first sensor and the second sensor are mounted to be irradiated to the sheet at different incident angles.,

21. A well plate sealing apparatus, comprising:
a well plate loading area in which the well plate is loaded on a tray and unloaded from the tray;
a sheet supply area in which the adsorbed sheet, the uppermost sheet among the stacked sheets in a magazine, is transferred and supplied on the well plate;
a heating area for applying heat to the sheet covered on the well plate; and
a tray transfer unit for transferring the tray on which the well plate is loaded along the loading area, the sheet supply area, and the heating area.

22. The well plate sealing apparatus according to claim 21,
further comprising a pickup unit provided with a hand for adsorbing the sheet and at least one blower fan positioned behind the hand, and configured to place the adsorbed sheet down on the well plate in the sheet supply area.

23. The well plate sealing apparatus according to claim 22,
wherein the pickup unit configured to operate at least one of the following operations: a first operation, in which the blower fan is driven to generate positive pressure in the hand to separate the sheet adsorbed to the hand, and a second operation, in which the blower fan is driven to generate negative pressure in the hand to adsorb the uppermost sheet.

24. The well plate sealing apparatus according to claim 23,
wherein the blower fan comprises a first blower fan configured to generate positive pressure in the hand and a second blower fan configured to generate negative pressure in the hand, and
wherein the first blower fan and the second blower fan are arranged side by side in a direction perpendicular to the sheet.

25. The well plate sealing apparatus according to claim 23,
further comprising at least one blower fan configured to suction air from the sheet supply area and discharge the air to the heating area.

26. The well plate sealing apparatus according to claim 22,
further comprising the magazine configured to accommodate the stacked sheets, and a magazine guide rail configured to guide the magazine in a direction inclined vertically,
wherein the magazine is descended along the magazine guide rail by gravity and is stopped at a predetermined sheet pick-up position.

27. The well plate sealing apparatus according to claim 26,
further comprising a sheet transfer unit comprising a rotation driving part configured to rotatably drive a hand picking up the sheet between the magazine and the well plate, and a translation driving part configured to drive the hand to be translatable between the magazine and the well plate.

28. The well plate sealing apparatus according to claim 21,
further comprising a pickup unit configured to pick up the uppermost sheet of the sheets in a magazine configured to accommodate sheets on which film sheet and slip sheet are alternately stacked,
wherein the sheet feed area comprises a placing position where the pickup unit places the film sheet down on the well plate disposed above the tray when the picked-up sheet is the film sheet,
wherein the well plate is moved to the placing position and the first sheet is placed down on the well plate when the picked-up sheet by the pickup unit is the first sheet, and
wherein the well plate is moved out of the placing position and the second is placed down outside the well plate when the picked-up sheet by the pickup unit is the second sheet.

29. The well plate sealing apparatus according to claim 1,
further comprising:
a tray transfer unit configured to transfer the tray;
a sheet sensor configured to sense the sheet accommodated in the magazine;
a well plate sensor configured to sense whether the well plate is disposed on the tray; and
a controller,
wherein the controller is configured to:
a) operate the tray transfer unit to transfer the tray to the placing position when it is determined that the well plate is disposed on the tray by receiving a signal from the well plate sensor,
b) operate the pickup unit to pick up the sheet when it is determined that the sheet is accommodated in the magazine by receiving a signal from the sheet sensor,
c) operate the sheet transfer unit to transfer the sheet to the placing position,
d) operate the pickup unit to drop the sheet on the well plate,
e) operate the tray transfer unit to move the tray to a sheet sealing area after the d),
f) operate the sealing unit to seal wells of the well plate with the sheet after the e), and
g) operate the tray transfer unit to transfer the tray to the loading are after the f).

30. The well plate sealing apparatus according to claim 29,
wherein the pickup unit is configured to pick up the uppermost sheet among the plurality of sheets in the magazine configured to accommodate sheets on which the film sheet and the slip sheet are alternately stacked,
the well plate sealing apparatus further comprises a sheet sensor configured to sense each of the film sheet and the slip sheet, and
wherein in the step d),
d1) the pickup unit is operated to place the film sheet down on the well plate waiting at the placing position when it is determined that the picked-up sheet by the pickup unit is the film sheet by using a signal of the sheet sensor, and
d2) the tray transfer unit is operated to move the tray to a position deviated from the placing position, and the pickup unit is operated to place the slip sheet down at the placing position when it is determined that the picked-up sheet by the pickup unit is the slip sheet by receiving the signal of the sheet sensor.

31. The well plate sealing apparatus according to claim 29,
wherein the pickup unit comprises a hand configured to hold a sheet, and at least one blower fan positioned behind the hand, and
wherein the blower fan comprises a first blower fan configured to generate positive pressure in the hand, and a second blower fan configured to generate negative pressure in the hand.

32. The well plate sealing apparatus according to claim 15,
wherein the magazine comprises a sheet separation part configured to provide an external force to separate the uppermost sheet of the sheets picked up by the pickup unit and a sheet thereunder,
wherein the sheet separation part comprises a first friction member provided on an inner wall of one side of the magazine, and
wherein the hand is configured to rotate at a predetermined angle about a rotation axis parallel to the plane direction of the sheet in a state in which the uppermost sheet is picked up, to rub the picked-up sheet against the first friction member.

33. The well plate sealing apparatus according to claim 32,
wherein the first friction member is provided at one side in the longitudinal direction of the magazine, and
wherein the hand is configured to rotate at a predetermined angle about a rotation axis parallel to the width direction of the sheet, to rub the picked-up sheet against the first friction member.

34. The well plate sealing apparatus according to claim 15,
wherein the magazine comprises a sheet separation part configured to provide an external force to separate the uppermost sheet of the sheets picked up by the pickup unit and a sheet thereunder,
wherein the sheet separation part comprises a second friction member provided on an inner surface of a side wall in the width direction of the magazine, and
wherein the hand is configured to move in the height direction having a predetermined angle with respect to the plane direction of the sheet in a state in which the uppermost sheet is picked up, to rub the picked-up sheet against the second friction member.

35. The well plate sealing apparatus according to claim 15,
wherein the pickup unit further comprises a contact sensor configured to sense whether the hand has contacted the sheet, and
the well plate sealing apparatus further comprises a controller,
wherein the controller is configured to:
a) operates the pickup unit to approach the sheet in a state in which a pick-up surface of the hand is parallel to the stacked sheets,
b) operates the pickup unit to pick up the uppermost sheet when it is determined the pick-up surface of the hand has contacted the sheet by using a signal of the contact sensor,
c) moves the hand away from the stacked sheets by a predetermined distance after the hand picks up the uppermost sheet,
d) rotates the hand by a predetermined angle in a one direction about a rotation axis parallel to the width direction of the sheet,
e) rotates the hand by a predetermined angle in a reverse direction about a rotation axis to approach the sheet in a state in which the pick-up surface is parallel to the stacked sheets,
f) rotates the hand by a predetermined angle in the reverse direction about a rotation axis, and
g) rotates the hand by a predetermined angle in the one direction about a rotation axis so that the pick-up surface is parallel to the stacked sheets.

36. The well plate sealing apparatus according to claim 35,
wherein the magazine comprises a sheet separation part configured to provide an external force to separate the uppermost sheet of the sheets picked up by the pickup unit and a sheet thereunder,
wherein the sheet separation part comprises a first friction member provided on an inner wall of one side in the longitudinal direction of the magazine, and
wherein the hand is rotated about the rotation shaft at a predetermined angle so that the picked-up sheet is rubbed against the first friction member.

37. The well plate sealing apparatus according to claim 36,
wherein the sheet separation part further comprises a second friction member provided on an inner wall of one side in the width direction of the magazine, and
wherein the controller controls the hand to move at least one movement of back and forward a predetermined distance from the stacked sheets so that the picked-up sheet is rubbed against the second friction member after the hand picks up the uppermost sheet

38. A sheet transfer apparatus comprising:
a magazine configured to accommodate stacked sheets;
a pickup unit configured to pick up a uppermost sheet of the sheets in the magazine;
a tray on which a target member is disposed;
a sheet transfer unit coupled to the pickup unit and configured to transfer the picked-up sheet to a placing position; and
a controller,
wherein the placing position is a position at which the picked-up sheet is placed on the target member disposed on the tray,
wherein the pickup unit comprises a hand configured to hold the sheet and at least one blower fan positioned behind the hand, and
wherein the controller controls the fan to be driven so that negative pressure is generated in the hand to hold the uppermost sheet of the sheets in the magazine.

39. A sheet transfer apparatus comprising:
a magazine configured to accommodate stacked sheets;
a pickup unit configured to pick up the uppermost sheet among the sheets in the magazine; and
a controller,
wherein the pickup unit comprises a hand configured to pick up the sheet, and
wherein the controller controls the hand to perform a rubbing motion in a state in which the hand is in contact with the uppermost sheet to separate the uppermost sheet from the underlying sheet.

40. The well plate sealing apparatus according to claim 1,
wherein the magazine is configured to accommodate sheets on which the film sheet and the slip sheet are alternately stacked,
wherein the pickup unit comprises a hand configured to pick up the sheets and a sheet sensor configured to detect each of the film sheet and the slip sheet, and
wherein the placing position is a position at which the pickup unit places the film sheet down on the well plate disposed on the tray when the picked-up sheet is the film sheet.

41. A sheet adsorbing apparatus comprising:
a magazine configured to accommodate stacked sheets;
a pickup unit configured to pick up the uppermost sheet of the sheets in the magazine; and
a controller,
wherein the pickup unit comprises a hand configured to adsorb the sheets and a blower fan positioned behind the hand,
wherein the controller controls the fan to be driven so that negative pressure is generated in the hand to adsorb the uppermost sheet of the sheets in the magazine, and determines whether the sheet is adsorbed on the hand by receiving a signal including driving information of the blower fan or pressure information behind the hand after driving the blower fan.

42. The well plate sealing apparatus according to claim 1,
wherein the tray is configured to disposed with a well plate including a plurality of wells arranged in a first direction,
the well plate sealing apparatus further comprises: a tray transfer unit comprising a tray transfer rail that guide the tray in the first direction and a tray driving unit, and a control unit,
wherein the pickup unit comprises: a hand configured to pick up the sheet; and a sheet sensor configured to sense whether the sheet is present on the well plate, and
wherein the controller controls:
i) the pickup unit to be driven to pick up the sheet,
ii) the pickup unit and the sheet transfer unit to be driven to place the sheet down on the well plate,
iii) the pickup unit and the tray relatively to move in the first direction,
iv) sensing whether the sheet is present using the sheet sensor at a plurality of predetermined positions with respect to an expected position of the well plate on the tray transfer rail, and
v) determining whether the sheet is positioned in a normal range on the well plate in the first direction through a signal of the sheet sensor.
